# EUROPEAN PATENT APPLICATION

(11) **EP 1 600 159 A1**
(43) Date of publication of application: **30.11.2005**
(21) Application number: 05016275.9
(22) Date of filing: 24.04.2001
(51) Int. Cl.: A61K 31/44, C07D 471/02, A61P 43/00, C07D 471/04

(54) **A method for synthesizing different Zolpidem hemitartrate derivatives**

(30) Priority: 24.04.2000 US 199298 P; 22.05.2000 US 206025 P; 14.08.2000 US 225364 P
(62) Divisional of application: 01930705.7
(71) Applicant: Teva Pharmaceutical Industries Ltd, 49131 Petah Tiqva (IL)
(72) Inventor: Aronhime, Judith, 76217 Rechovot (IL); Salyi, Szaboles, 4031 Debrecen (HU); Leonov, David, Rechovot (IL); Szabo, Csaba, 4031 Debrecen (HU); Meszaros-Sos, Erzebet, 4031 Debrecen (HU); Zavurov, Shlomo, Lod (IL)
(74) Representative: Gallagher, Kirk James

(57) **Abstract**

The present invention provides a method for synthesizing novel forms of zolpidem hemitartrate.

## Description

### CROSS-REFERENCE TO RELATED APPLICATION

This invention claims the benefit under 35 U.S.C. 1.119(e) of provisional applications Serial Nos. 60/199,298, filed April 24, 2000; 60/206,025, filed May 2, 2000 and 60/225,364, filed August 14, 2000.

### FIELD OF THE INVENTION

The present invention relates to novel hydrate, anhydrous and solvate crystal forms of zolpidem hemitartrate and the preparation thereof.

### BACKGROUND OF THE INVENTION

Zolpidem, as a hemitartrate salt, is currently approved for the short-term treatment of insomnia in the United States under the trademark of AMBIEN. Zolpidem hemitartrate is classified as a non-benzodiazepine hypnotic of the imidazopyridine class. It has little effect on the stages of sleep in normal human subjects and is as effective as benzodiazepines in shortening sleep latency and prolonging total sleep time in patients with insomnia. The development of tolerance and physical dependence for patients using AMBIEN has been seen only very rarely and under unusual circumstances. (Goodman and Gilman's, *The Pharmacological Basis of Therapeutics* 371 (Joel G. Hardman et al., eds. 9th ed. 1996)).

Zolpidem hemitartrate (CAS Registry No. 99294-93-6) has the chemical name imidazo[1,2-a]pyridine-3-acetamide,N,N,6-trimethyl-2-(4-methylphenyl)-,(2R,3R)-2,3-di hydroxy-butanedioate and is represented by the structural formula.

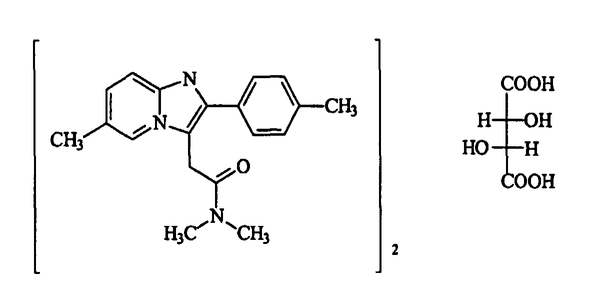

Zolpidem is among the compounds described in the following U.S. patents which are incorporated herein by reference: 4,382,938; 4,794,185; 4,356,283; 4,460,592; 4,501,745; 4,675,323; 4,808,594; and 4,847,263. The above US Patents disclose Zolpidem as having, inter alia, anxiolytic, sleep-inducing, hypnotic and anticonvulsant properties.

### SUMMARY OF THE INVENTION

The present invention provides for a zolpidem hemitartrate hydrate.

In an alternative embodiment, the present invention provides a zolpidem hemitartrate monohydrate.

In an alternative embodiment, the present invention provides a zolpidem hemitartrate dihydrate.

In an alternative embodiment, the present invention provides a zolpidem hemitartrate trihydrate.

In an alternative embodiment, the present invention provides a zolpidem hemitartrate tetrahydrate.

In an alternative embodiment, the present invention provides a zolpidem hemitartrate solvate.

In an alternative embodiment, the present invention provides zolpidem hemitartrate anhydrous.

In an alternative embodiment, the present invention provides zolpidem hemitartrate with not more than 1% water content.

In an alternative embodiment, the present invention provides zolpidem hemitartrate Form C.

In an alternative embodiment, the present invention provides zolpidem hemitartrate Form C, characterized by an X-ray powder diffraction pattern having peaks at about 7.3, 9.5, 17.8 and 23.8 ± 0.2 degrees two-theta.

In an alternative embodiment, the present invention provides a method for treating a patient suffering from insomnia by administering a therapeutically effective amount of the zolpidem hemitartrate Form C.

In an alternative embodiment, the present invention provides zolpidem hemitartrate Form D monohydrate.

In an alternative embodiment, the present invention provides zolpidem hemitartrate Form D, characterized by a water content of about 2.3 % to about 2.7 % by weight.

In an alternative embodiment, the present invention provides zolpidem hemitartrate Form D hemiethanolate.

In an alternative embodiment, the present invention provides zolpidem hemitartrate Form D, characterized by an X-ray powder diffraction pattern having peaks at about 7.1, 9.5, 14.1, 19.6 and 24.5 ±0.2 degrees two-theta.

In an alternative embodiment, the present invention provides a method for treating a patient suffering from insomnia by administering a therapeutically effective amount of zolpidem hemitartrate Form D.

In an alternative embodiment, the present invention provides zolpidem hemitartrate Form E dihydrate.

In an alternative embodiment, the present invention provides zolpidem hemitartrate Form E trihydrate.

In an alternative embodiment, the present invention provides zolpidem hemitartrate Form E tetrahydrate.

In an alternative embodiment, the present invention provides zolpidem hemitartrate Form E, characterized by a water content from about 5.0% to about 8.5% by weight.

In an alternative embodiment, the present invention provides zolpidem hemitartrate Form E, characterized by an X-ray powder diffraction pattern having peaks at about 5.2, 7.9, 10.4, 17.2, 18.0 and 18.8 ±0.2 degrees two-theta.

In an alternative embodiment, the present invention provides a method for treating a patient suffering from insomnia by administering a therapeutically effective amount of the zolpidem hemitartrate Form E.

In an alternative embodiment, the present invention provides zolpidem Form F methanolate.

In an alternative embodiment, the present invention provides zolpidem hemitartrate Form F, characterized by a methanol content of about 5.5% by weight.

In an alternative embodiment, the present invention provides zolpidem hemitartrate Form F, characterized by an X-ray powder diffraction pattern having peaks at about 7.6 and 18.0 ± 0.2 degrees two-theta.

In an alternative embodiment, the present invention provides a method for treating a patient suffering from insomnia by administering a therapeutically effective amount of the zolpidem hemitartrate Form F.

In an alternative embodiment, the present invention provides zolpidem hemitartrate Form G solvate.

In an alternative embodiment, the present invention provides zolpidem hemitartrate Form G, characterized by an X-ray powder diffraction pattern having peaks at about 6.8 ± 0.2 degrees two-theta.

In an alternative embodiment, the present invention provides a method for treating a patient suffering from insomnia by administering a therapeutically effective amount of the zolpidem hemitartrate Form G.

In an alternative embodiment, the present invention provides zolpidem hemitartrate Form H, characterized by an X-ray powder diffraction pattern having peaks at about 7.7, 17.4, 18.0 and 24.3 ±0.2 degrees two-theta.

In an alternative embodiment, the present invention provides a method for treating a patient suffering from insomnia by administering a therapeutically effective amount of the zolpidem hemitartrate Form H.

In an alternative embodiment, the present invention provides zolpidem hemitartrate form L dihydrate.

In an alternative embodiment, the present invention provides zolpidem hemitartrate Form L, characterized by a water content of about 4.3% by weight.

In an alternative embodiment, the present invention provides zolpidem hemitartrate Form L, characterized by an X-ray powder diffraction pattern having peaks at about 6.8, 9.7, 17.3, 19.6 and 21.1 ±0.2 degrees two-theta.

In an alternative embodiment, the present invention provides a method for treating a patient suffering from insomnia by administering a therapeutically effective amount of the zolpidem hemitartrate Form L.

In an alternative embodiment, the present invention provides a method for synthesizing zolpidem hemitartrate, comprising the steps of: (a) forming a zolpidic acid halide from the zolpidic acid; (b) reacting zolpidem acid halide, with dimethyl amine, to form zolpidem base; (c) forming zolpidem hemitartrate salt from the zolpidem base.

In an alternative embodiment, the present invention provides a process for preparing zolpidem hemitartrate Form C, comprising the steps of exposing zolpidem hemitartrate Form A to vapors of isopropyl alcohol.

In an alternative embodiment, the present invention provides a process for preparing zolpidem hemitartrate Form C, comprising the step of heating zolpidem hemitartrate to a temperature from about 70°C to about 150°C for a sufficient time to convert zolpidem hemitartrate to Form C.

In an alternative embodiment, the present invention provides a process for preparing zolpidem hemitartrate Form D, comprising the step of exposing zolpidem hemitartrate Form A to water vapor at a relative humidity from about 60% to about 100%.

In an alternative embodiment, the present invention provides a process for preparing zolpidem hemitartrate Form D, comprising the step of exposing Form C to water vapor at a relative humidity of about 100%.

In an alternative embodiment, the present invention provides a process for preparing zolpidem hemitartrate Form D, comprising the step of exposing zolpidem hemitartrate Form A to vapors of ethanol.

In an alternative embodiment, the present invention provides a process for preparing zolpidem hemitartrate Form D, comprising the step of exposing zolpidem hemitartrate Form C to vapors of ethanol.

In an alternative embodiment, the present invention provides a process for preparing zolpidem hemitartrate Form D, comprising the step of forming a slurry of zolpidem hemitartrate Form A in ethylacetate.

In an alternative embodiment, the present invention provides a process for preparing zolpidem hemitartrate Form D, comprising the step of forming a slurry of zolpidem hemitartrate Form A in acetone.

In an alternative embodiment, the present invention provides a process for preparing zolpidem hemitartrate Form D, comprising the step of granulating zolpidem hemitartrate Form A in isopropanol.

In an alternative embodiment, the present invention provides a process for preparing zolpidem hemitartrate Form C, comprising the step of forming a slurry of zolpidem hemitartrate Form A in isopropanol.

In an alternative embodiment, the present invention provides a process for preparing zolpidem hemitartrate Form D, comprising the step of granulating zolpidem hemitartrate Form A in butanol.

In an alternative embodiment, the present invention provides a process for preparing zolpidem hemitartrate Form E, comprising the step of exposing a solid form of zolpidem hemitartrate to water vapor at a relative humidity of about 100%.

In an alternative embodiment, the present invention provides a process for preparing zolpidem hemitartrate Form E, comprising the step of forming a slurry of a solid form of zolpidem hemitartrate in water.

In an alternative embodiment, the present invention provides a process for preparing zolpidem hemitartrate Form E, comprising the step of granulating a solid form of zolpidem hemitartrate in water.

In an alternative embodiment, the present invention provides a process for preparing zolpidem hemitartrate Form F, comprising the step of exposing a solid form of zolpidem hemitartrate to vapors of methanol.

In an alternative embodiment, the present invention provides a process for preparing zolpidem hemitartrate Form G, comprising the step of exposing zolpidem hemitartrate Form A to vapors of ethyl acetate.

In an alternative embodiment, the present invention provides a process for preparing zolpidem hemitartrate Form G, comprising the step of forming a slurry of zolpidem hemitartrate Form C in ethanol.

In an alternative embodiment, the present invention provides a process for preparing zolpidem hemitartrate Form G, comprising the step of forming a slurry of zolpidem hemitartrate Form C in methanol.

In an alternative embodiment, the present invention provides a process for preparing zolpidem hemitartrate Form G, comprising the step of granulating zolpidem hemitartrate Form C in ethanol.

In an alternative embodiment, the present invention provides a process for preparing zolpidem hemitartrate Form G, comprising the step of granulating zolpidem hemitartrate Form C in methanol.

In an alternative embodiment, the present invention provides a process for preparing zolpidem hemitartrate Form H, comprising the step of slurrying zolpidem hemitartrate Form A in ethanol.

In an alternative embodiment, the present invention provides a process for preparing zolpidem hemitartrate Form H, comprising the step of slurrying zolpidem hemitartrate Form A in methanol.

In an alternative embodiment, the present invention provides a process for preparing zolpidem hemitartrate Form H, comprising the step of granulating zolpidem hemitartrate Form A in ethanol.

In an alternative embodiment, the present invention provides a process for preparing zolpidem hemitartrate Form H, comprising the step of granulating zolpidem hemitartrate Form A in methanol.

In an alternative embodiment, the present invention provides a process for preparing zolpidem hemitartrate Form L, comprising the step of: (a) dissolving zolpidem hemitartrate in a solvent mixture of methanol and water; (b) precipitating zolpidem hemitartrate from the solvent mixture; and, (c) isolating zolpidem hemitartrate.

In an alternative embodiment, the present invention provides a zolpidem hemitartrate having particles up to about 200 microns in size.

In an alternative embodiment, the present invention provides a zolpidem hemitartrate having particles up to about 50 microns in size.

In an alternative embodiment, the present invention provides a pharmaceutical composition comprising a therapeutically effective amount of zolpidem hemitartrate particles up to about 200 microns in size as measured by laser diffraction and, a pharmaceutically acceptable carrier.

In an alternative embodiment, the present invention provides a pharmaceutical composition of claim 118, wherein the zolpidem hemitartrate particles are selected from the group consisting of Form A, Form B, Form C, Form D, Form E, Form F, Form G, Form H and Form L.

In an alternative embodiment, the present invention provides a pharmaceutical composition comprising a therapeutically effective amount of zolpidem hemitartrate particles up to about 50 microns in size as measured by laser diffraction and, a pharmaceutically acceptable carrier.

In an alternative embodiment, the present invention provides a pharmaceutical composition of claim 120, wherein the zolpidem hemitartrate particles are selected from the group consisting of Form A, Form B, Form C, Form D, Form E, Form F, Form G, Form H and Form L.

In an alternative embodiment, the present invention provides a micronized zolpidem hemitartrate Form A having particles up to about 200 microns in size as measured by laser diffraction and an x-ray diffraction pattern having a peak at about 8.6 ±0.2 degrees two-theta.

### BRIEF DESCRIPTION OF THE FIGURES

Fig. 1. shows the X-ray diffraction pattern of zolpidem hemitartrate Form A.
Fig. 2. shows the DTG thermal profile of zolpidem hemitartrate Form A.
Fig. 3. shows the X-ray diffraction pattern of zolpidem hemitartrate Form C.
Fig. 4. shows the DTG thermal profile of zolpidem hemitartrate Form C.
Fig. 5. shows the X-ray diffraction pattern of zolpidem hemitartrate Form D.
Fig. 6. shows the DTG thermal profile of zolpidem hemitartrate Form D.
Fig. 7. shows the X-ray diffraction pattern of zolpidem hemitartrate Form E.
Fig. 8. shows the DTG thermal profile of zolpidem hemitartrate Form E.
Fig. 9. shows the X-ray diffraction pattern of zolpidem hemitartrate Form F.
Fig. 10. shows the X-ray diffraction pattern of zolpidem hemitartrate Form G.
Fig. 11. shows the DTG thermal profile of zolpidem hemitartrate Form G.
Fig. 12. shows the X-ray diffraction pattern of zolpidem hemitartrate Form H.
Fig. 13. shows the DTG thermal profile of zolpidem hemitartrate Form H.
Fig. 14. shows the X-ray diffraction pattern of zolpidem hemitartrate Form L.
Fig. 15. shows the DTG thermal profile of zolpidem hemitartrate Form L.
Fig. 16. shows an X-Ray diffraction pattern for micronized Form A.

### DETAILED DESCRIPTION OF THE INVENTION

The present invention provides new hydrate, anhydrous and solvate crystal forms of zolpidem hemitartrate. Crystal forms of a compound can be distinguished in a laboratory by X-ray diffraction spectroscopy and by other methods such as, infrared spectrometry. It is desirable to investigate all solid state forms of a drug, including all crystal/polymorphic forms, and to determine the stability, dissolution and flow properties of each crystal/polymorphic form. For a general review of polymorphs and the pharmaceutical applications of polymorphs see G.M. Wall, *Pharm Manuf*. **3,** 33 (1986); J.K. Haleblian and W. McCrone, *J. Pharm. Sci.,* **58,** 911 (1969); and J.K. Haleblian, *J. Pharm. Sci.,* **64,** 1269 (1975), all of which are incorporated herein by reference.

As used herein, the term "zolpidem hemitartrate" includes hydrates and solvates of zolpidem hemitartrate. The term "water content" refers to the content of water based upon the Loss on Drying method (the "LOD" method) as described in Pharmacopeial Forum, Vol. 24, No. 1, p. 5438 (Jan - Feb 1998), the Karl Fisher assay for determining water content or thermogravimetric analysis (TGA). The term "equivalents of water" means molar equivalents of water. All percentages herein are by weight unless otherwise indicated. Those skilled in the art will also understand that the term "anhydrous" when used in reference to zolpidem hemitartrate describes zolpidem hemitartrate which is substantially free of water. Those skilled in the art will appreciate that the term "monohydrate" when used in reference to zolpidem hemitartrate describes a crystalline material having a water content of about 2.3% w/w. One skilled in the art will also appreciate that the term "dihydrate" when used in reference to zolpidem hemitartrate describes a crystalline material having a water content of about 4.5% w/w. One skilled in the art will also appreciate that the term "trihydrate" when used in reference to zolpidem hemitartrate describes a crystalline material having a water content of about 6.6% w/w. One skilled in the art will also appreciate that the term "tetrahydrate" when used in reference to zolpidem hemitartrate describes a crystalline material having a water content of about 8.6% w/w. One skilled in the art will also appreciate that the term "methanolate","ethanolate", "solvates of isopropranol", "solvates of butanol" or "solvates of ethylacetate" refers to Zolpidem hemitartrate in which solvent is contained within the crystal lattice in quantities above 1%. One skilled in the art will also appreciate that the term "hemiethanolate" when used in reference to zolpidem hemitartrate describes a crystalline material having an ethanol content of about 2.9% w/w.

Hydrate and solvate forms of zolpidem hemitartrate are novel and distinct from each other in terms of their characteristic powder X-ray diffraction patterns and their thermal profiles.

For the purposes of this specification, ambient temperature is from about 20°C to about 25°C.

All powder X-ray diffraction patterns were obtained by methods known in the art using a Scintag X'TRA X-ray powder diffractometer, equipped with a solid stae Si(Li) detector thermoelectrically cooled, at scanning speed of 3° min.⁻¹ scanning range 2-40 degrees two-theta. Copper radiation of λ = 1.5418 Å was used.

Measurement of thermal analysis are conducted for the purpose of evaluating the physical and chemical changes that may take place in a heated sample. Thermal reactions can be endothermic (eg, melting, boiling, sublimation, vaporization, desolvation, solid-solid phase transitions, chemical degradation, etc.) or exothermic (eg, crystallization, oxidative decomposition, etc.) in nature. Such methodology has gained widespread use in the pharmaceutical industry in characterization of polymorphism. The quantitative applications of thermal applications of thermal analysis have proven to be useful in characterization of polymorphic systems. The most commonly applied techniques are thermogravimetry (TGA), differential thermal analysis (DTA), and differential scanning calorimetry (DSC).

The DTA and TGA curves presented herein were obtained by methods known in the art using a DTG Shimadzu model DTG-50 (combined TGA and DTA). The weight of the samples was about 9 to about 13 mg. The samples were scanned up to about 300°C or above at a rate of 10°C/min. Samples were purged with nitrogen gas at a flow rate of 20 ml/min. Standard alumina crucibles covered lids with one hole.

Thermogravimetry analysis (TGA) is a measure of the thermally induced weight loss of a material as a function of the applied temperature. TGA is restricted to transitions that involve either a gain or a loss of mass, and it is most commonly used to study desolvation processes and compound decomposition.

Karl Fisher analysis, which is well known in the art, is also used to determine the quantity of water in a sample.

As used herein a slurry refers to a suspension of insoluble particles or slightly soluble particles in an aqueous or organic (non-aqueous) liquid, without complete dissolution of the solid.

### SYNTHESIS OF ZOLPIDEM HEMITARTRATE

The present invention provides a methods of synthesizing zolpidem hemitartrate. Zolpidem hemitartrate base may be synthesized as disclosed in U.S. Patent No. 4,382,938. Therein, is disclosed that zolpidem base may be formed from zolpidic acid by reacting the acid with dimethyl amine, in the presence of carbonyldiimidazole. This method has several disadvantages such as low yield and formation of impurities which are difficult to remove. US Patent No. 4,382,938 also mentions the possibility of reacting zolpidic acid chloride with dimethyl amine. However no procedure for this preparation of zolpidiem is mentioned. The present patent present a procedure for the preparation of Zolpidem from zolpidic acid which has the following advantages:
- High reaction yields
- Improved purity profile of the prepared Zolpidem
- Preparation of Zolpidem from zolpidic acid in a "one pot " procedure.
Alternatively the base may be formed by reacting the zolpidic acid chloride with dimethyl amine. U.S. Patent No. 4,794,185 discloses alternative methods for synthesizing zolpidem base.

Once zolpidem base is formed, the zolpidem hemitartrate is prepared by dissolving the base in methanol and adding L(+)-tartric acid dissolved in methanol. The hemitartrate is then crystalized from methanol.

### Formation of the Acid Chloride

Preferably, formation of zolpidem base from the acid comprises a two-step reaction. In the first step, zolpidic acid chloride (II) is formed from zolpidic acid (I).

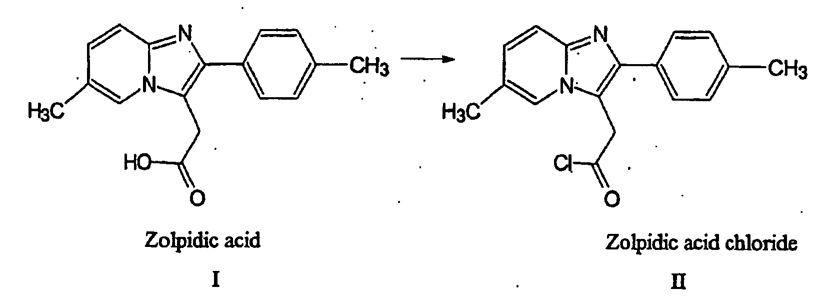

The chlorination reaction can be performed using SOCl₂, PCL₅ and POCL₃. The most preferred chlorination agent is SOCl₂ because its excess can be removed smoothly after the reaction end by distillation from the reaction mass.

Preferred solvents are aliphatic or aromatic hydrocarbons, chlorinated solvents and aprotic polar solvents and mixtures thereof. The most preferred reaction solvent is toluene containing traces of dimethylformamide (DMF). The use of toluene as reaction solvent has the following advantages:
1. The intermediate zolpidic acid chloride (II) and the final zolpidem can be isolated at whish from this solvent in such a way that the procedure can be designed as a one step or two step process.
2. In the presence of toluene the excess of SOCl₂ is easy to remove as an azeotrop SOCl₂- toluene.
3. Zolpidic acid chloride (II) precipitate from the reaction mixture during the chlorination reaction. An overchlorination of the zolpidic acid is thus avoided.

DMF can be regarded as a phase transfer catalyst of the chlorination reaction by facilitating the access of SOCl₂ to the Zolpidic acid Also in the precipitation of Zolpidem the presence of DMF contribute to a better purification effect of the reaction solvent.

The preferred temperature range for forming the acid chloride is from about 15 to about 28°C. Most preferably the temperature is in the range of about 18 to about 22 °C.

After formation of the acid chloride, the thionyl chloride is distilled from the reaction mixture.

The preferred temperature of distillation is in the range of about 30 to about 40 °C. Most preferably the temperature of distillation is in the range of about 35 to about 40 °C.

The pressure of vacuum distillation is in the range of about 30 to about 100 mm Hg. Most preferably the pressure is in the range of about 30 to about 50 mm Hg.

### Formation of Zolpidem Base

In the second step, zolpidic acid chloride (II) is used to form zolpidem base (the compound of formula III) in a reaction with dimethyl amine as shown.

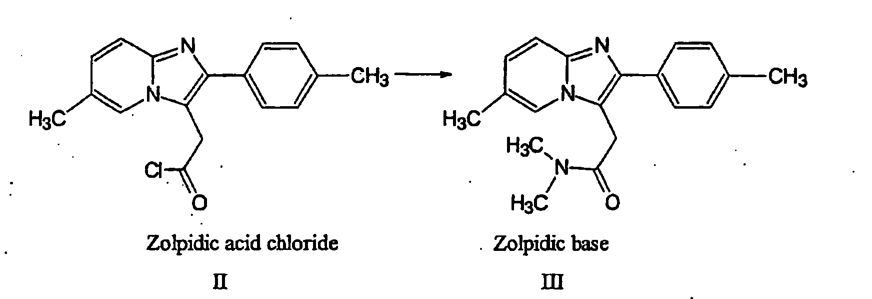

Preferred solvents are aliphatic or aromatic hydrocarbons, chlorinated solvents and aprotic polar solvents and mixtures thereof The most preferred solvent is toluene. The use of toluene as reaction solvent has the following advantages:
1. Zolpidic acid and coloured impurities formed during the chlorination reaction are effectively removed.
2. The crystallization process in toluene is optimal.
3. Essentially pure Zolpidem (98% area% by HPLC) is obtained

Dimethylamine is preferably introduced as a gas until the pH is from about 8.5 and about 9.5.

The preferred temperature range for forming the base is from about -5 to about +3 °C. Most preferably the temperature is in the range of about -5 to about 0 °C.

After dimethylamine gas is introduced, the resulting zolpidem base forms a precipitate. After the solution is mixed for 1 hour, the solution is cooled to about -10 to about -12 °C. Typically the precipitate is then collected by filtration. However, the precipitate may be collected by any means known in the art.

The zolpidem base is then dried and used to form the hemitartrate by dissolving the base in methanol and adding L(+)-tartric acid dissolved in methanol. The hemitartrate is then crystalized from methanol.

### NOVEL HYDRATE, ANHYDROUS AND SOLVATE FORMS OF ZOLPIDEM HEMITARTRATE

The present invention provides novel crystal forms of zolpidem hemitartrate which will be designated as Forms B, C, D, E, F, G, H and L. These forms can be distinguished from the prior art form of zolpidem hemitartrate and from each other by characteristic powder X-ray diffraction patterns and thermal profiles.

The different crystal forms may also be characterized by their respective solvation state. The most commonly encountered solvates among pharmaceuticals are those of 1:1 stoichiometry. Occasionally mixed solvate species are encountered. When water or solvent is incorporated into the crystal lattice of a compound in stoichiometric proportions, the molecular adduct or adducts formed are referred to as hydrates or solvates.

### Zolpidem hemitartrate Form A

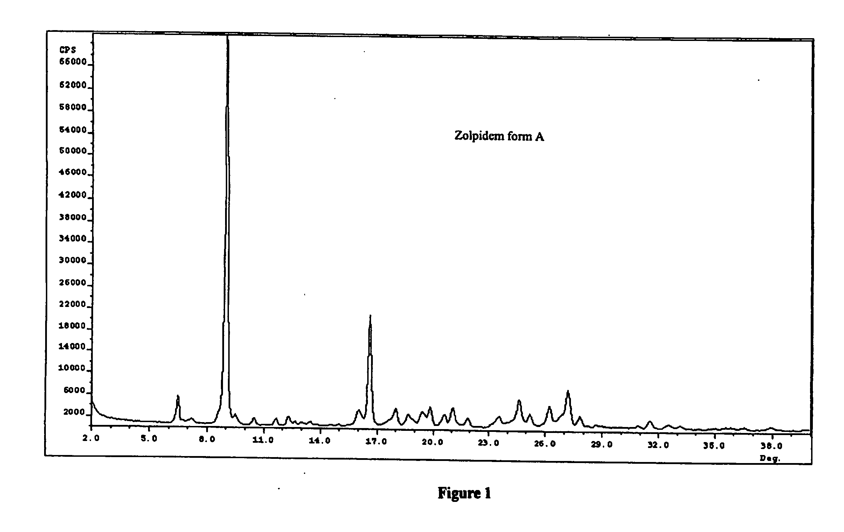

Zolpidem hemitartrate Form A ("Form A") is characterized by an X-ray diffraction pattern with peaks at about 6.5, 9.0, 16.1, 16.6, 24.6 and 27.3 ±0.2 degrees two-theta. The diffraction pattern is reproduced in Fig. 1. The above x-ray diffraction pattern was found in the prior art EP standard sample. When samples of From A containing substantially particles smaller than all 200 microns were examined the x-ray diffraction pattern showed a new peaks at about 8.6 ±0.2 degrees two-theta. Other unexpected peaks were observed at 6.7, 11.2, 15.4 and 17.3 ±0.2 degrees two-theta.

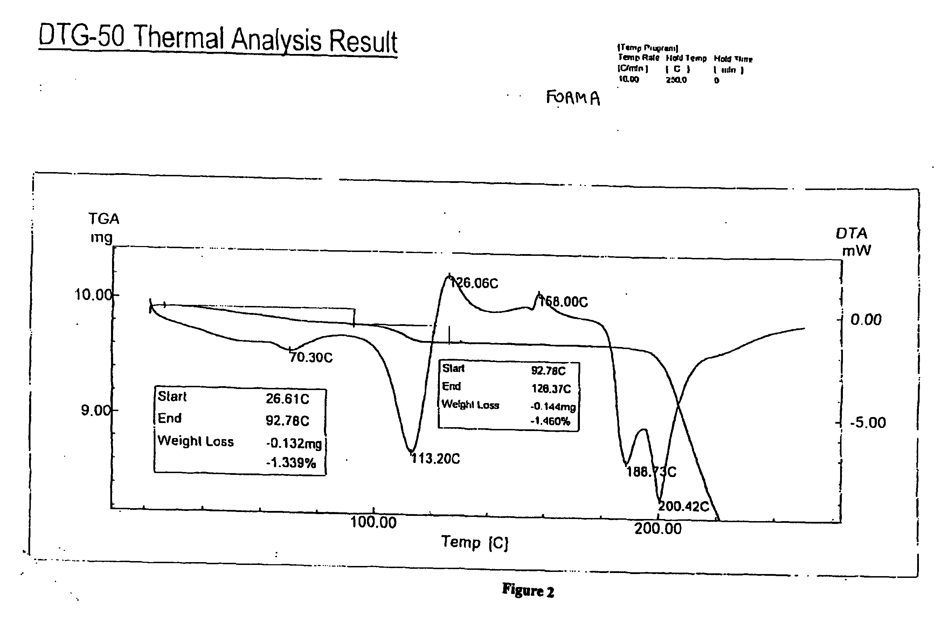

The DTG thermal profile of Form A is shown in Fig. 2. The thermal profile shows an endotherm at about 110°C, followed by an exotherm; an additional exothermic/endothermic event at above about 150°C; a melting endotherm at about 188°C; and an endothermic event at about 200°C concomitant to decomposition.

The hydration states of Form A is characterized by TGA and Karl Fisher analysis. Zolpidem hemitartrate described in the EP monograph (2001), herein identified as Form A, is reported as a hygroscopic solid. It was found by us, that Form A may contain about 1.0 % water or more, and readily absorbs up to 3.0 % water as measured by Karl Fischer analysis. The 110°C endotherm of the TGA is attributed to partial desorption of water with an overall water content of about 3%.

### Zolpidem hemitartrate Form B

Zolpidem hemitartrate Form B is characterized by a powder X-ray diffraction pattern at about 8.2, 17.3, and 18.4 ± 0.2 degrees two-theta.

### Zolpidem hemitartrate Form C

Zolpidem hemitartrate Form C ("Form C") is an anhydrous (i.e. non-solvated) form of crystalline zolpidem hemitartrate.

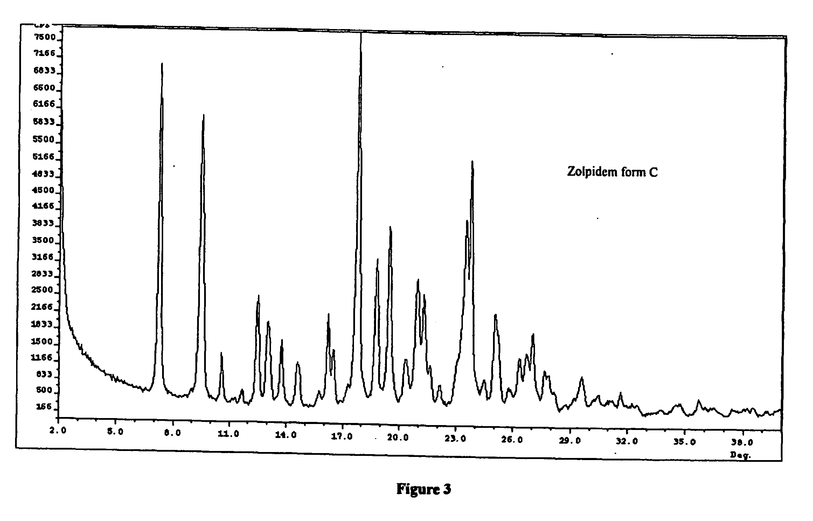

Zolpidem hemitartrate Form C is characterized by an X-ray diffraction pattern with peaks at about 7.3, 9.5, 10.7, 12.4, 13.0, 13.8, 14.6, 16.2, 17.8, 18.9, 19.5, 20.3, 21.3, 23.5, 23.8, 25.0, and 27.0 ± 0.2degrees two-theta. The most characteristic peaks of Form C are at about 7.3, 9.5, 17.8 and 23.8 ± 0.2degrees two-theta. The diffraction pattern is reproduced in Fig. 3.

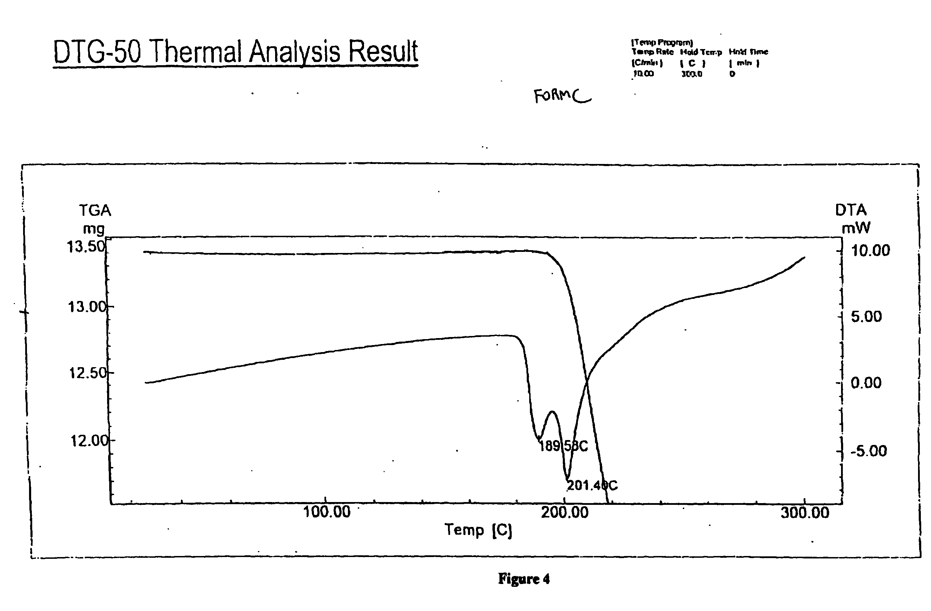

The DTG thermal profile of Form C is shown in Fig. 4. The thermal profile shows a melting endotherm at about 187°C and an endothermic event above 200°C concomitant to decomposition.

The unsolvated states of Form C is characterized by TGA and Karl Fisher analysis. The weight loss up to about 150°C (prior to decomposition) and the level of water measured by Karl Fischer are insignificant. Thus, the TGA and Karl Fisher analysis indicate that Form C is an anhydrous form of zolpidem hemitartrate.

### Zolpidem hemitartrate Form D

Zolpidem hemitartrate Form D ("Form D") is a monohydrate or hemiethanolate crystalline form of zolpidem hemitartrate.

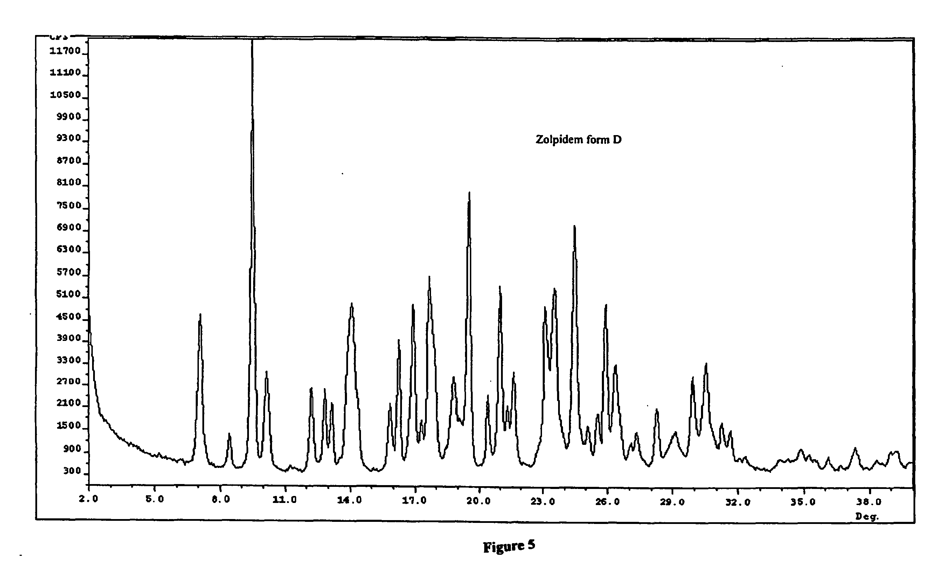

Zolpidem hemitartrate Form D is characterized by an X-ray diffraction pattern with peaks at about 7.1, 8.4, 9.5, 10.2, 12.2, 12.9, 13.2, 14.1, 15.9, 16.3, 17.7, 18.8, 19.6, 21.0, 21.7, 23.0, 23.6, 24.5, 25.9, 26.5, 30.0, and 30.6 ±0.2 degrees two-theta. The most characteristic peaks of Form D are at about 7.1, 9.5, 14.1, 19.6 and 24.5 ±0.2 degrees two-theta. The diffraction pattern is reproduced in Fig. 5.

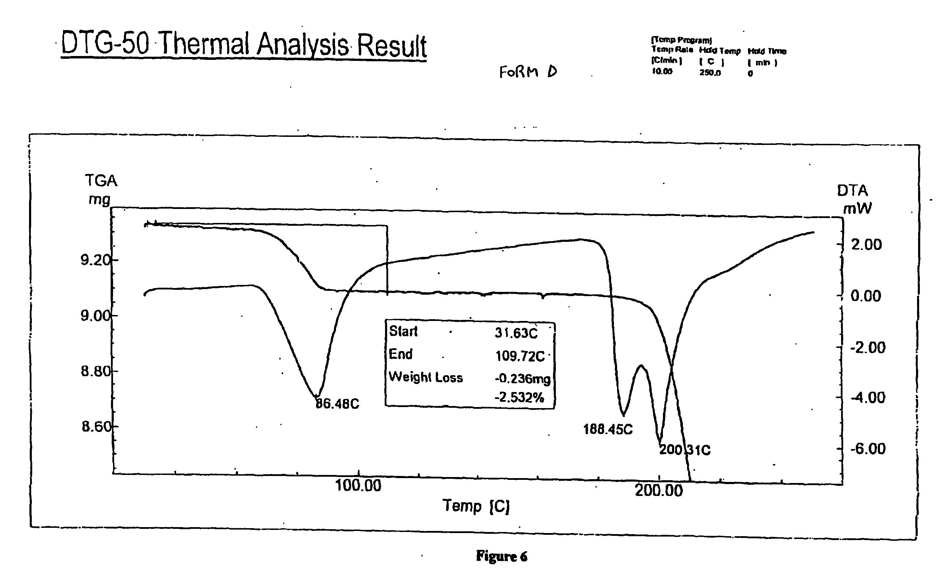

The DTG thermal profile of Form D is shown in Fig. 6. The DSC thermal profile shows an endotherm at about 80°C. In addition, a melting endotherm at 188°C and an endothermic event at about 200°C concomitant to decomposition occur.

The solvation states of Form D is characterized by TGA and Karl Fisher analysis. Form D has a weight loss of about 2.3 to about 2.7% by TGA (theoretical value of monohydrate: 2.3%, hemiethanolate: 2.9%) at about 80°C. The weight loss corresponds to a stoichiometric value of 1 or 1 1/4 mole of water per mole of zolpidem hemitartrate or to the stoichiometric value of hemiethanolate.

### Zolpidem hemitartrate Form E

Zolpidem hemitartrate Form E ("Form E") is a hydrate crystalline form of zolpidem hemitartrate which comprises dihydrate, trihydrate or tetrahydrate polymorphs.

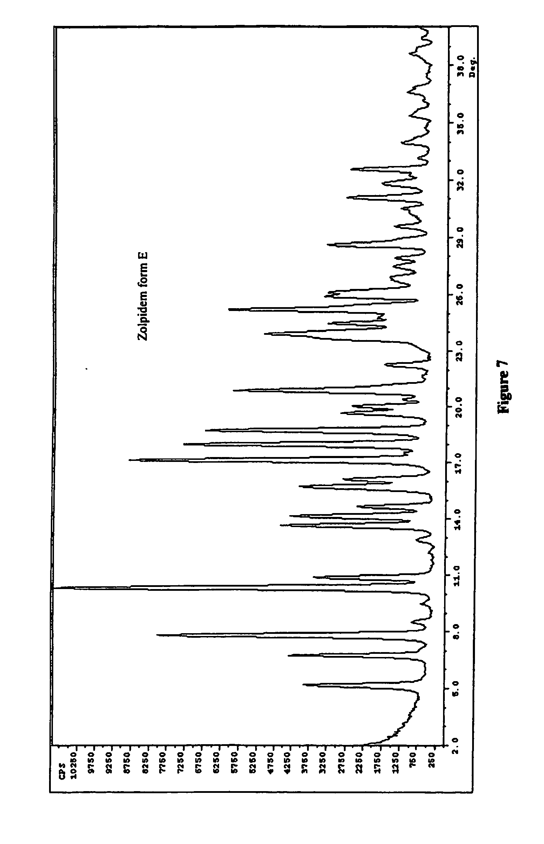

Zolpidem hemitartrate Form E is characterized by an X-ray diffraction pattern with peaks at about 5.2, 6.8, 7.9, 10.4, 11.0, 13.7, 14.2, 15.8, 16.1, 17.2, 18.0, 18.8, 19.7, 20.1, 22.2, 24.4, 25.2, 25.9, 28.5, 31.0, 31.8 and 32.5 ±0.2 degrees two-theta. The most characteristic peaks of Form E are at about 5.2, 7.9, 10.4, 17.2, 18.0 and 18.8 ±0.2 degrees two-theta. The diffraction pattern is reproduced in Fig. 7.

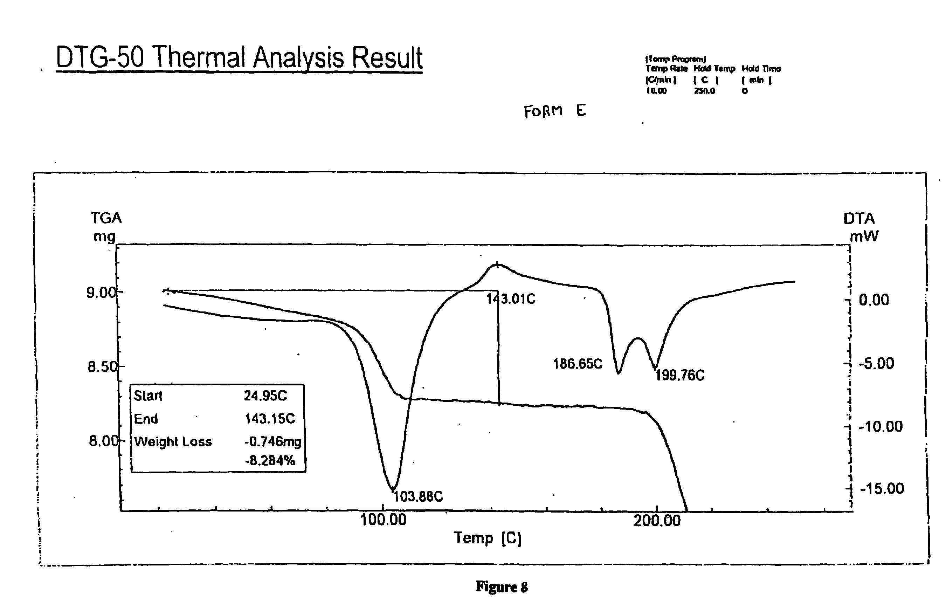

The DTG thermal profile of Form E is shown in Fig. 8. The DTG thermal profile of Form E contains a desorption endotherm with a peak maximum at about 100°C and a double endotherm of melting and decomposition at about 187°C and about 200°C.

The solvation states of Form E is characterized by TGA and Karl Fisher analysis. Form E has a weight loss of about 5.0 to about 8.5 % by TGA. The main weight loss occurs at about 90°C. The weight loss, corresponds to a stoichiometric value of 2, 3 or 4 molecules of water per molecule of zolpidem hemitartrate. The stoichiometry is confirmed by Karl Fischer analysis. (The theoretical value of the dihydrate is 4.5%, the trihydrate is 6.6%, the tetrahydrate is 8.6%).

### Zolpidem hemitartrate Form F

Zolpidem hemitartrate Form F ("Form F") is a methanolate crystalline form of zolpidem hemitartrate.

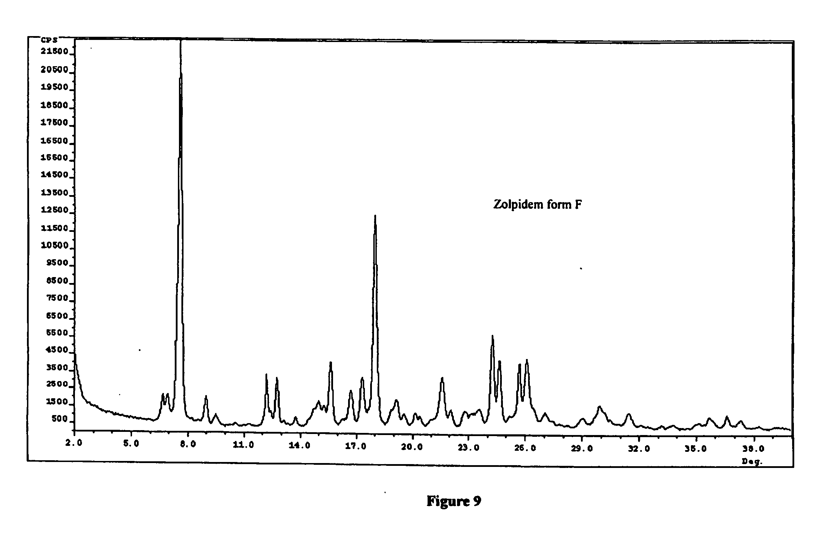

Zolpidem hemitartrate Form F is characterized by an X-ray diffraction pattern with peaks at about 7.6, 9.0, 12.2, 12.7, 15.7, 16.7, 17.3, 18.0, 19.6, 21.6, 24.3, 24.7, 25.7, and 26.1 ±0.2 degrees two-theta. The most characteristic peaks of Form F are at about 7.6 and 18.0 ±0.2 degrees two-theta. The diffraction pattern is reproduced in Fig. 9.

The solvation states of Form F is characterized by TGA and Karl Fisher analysis. Form F has a weight loss of about 5.5% corresponds to about 1½ methanol molecules per molecule of zolpidem hemitartrate.

### Zolpidem hemitartrate Form G

Zolpidem hemitartrate Form G ("Form G") is a solvate crystalline form of zolpidem hemitartrate.

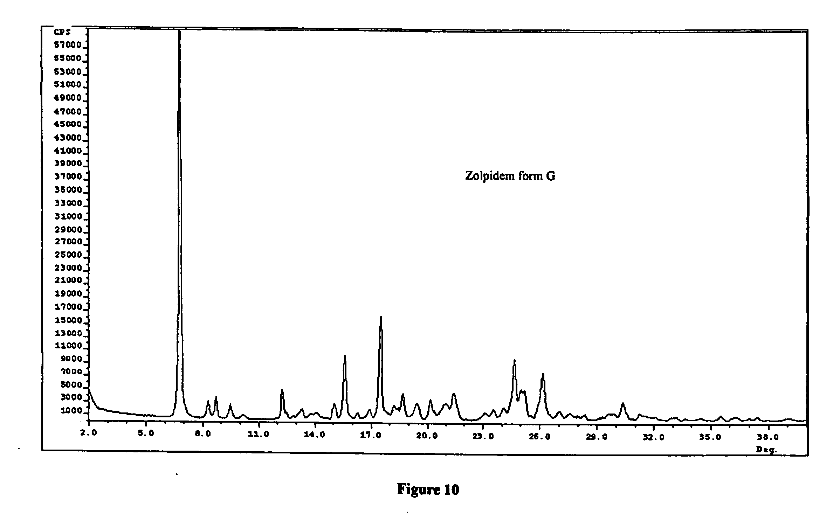

Zolpidem hemitartrate Form G is characterized by an X-ray diffraction pattern with peaks at about 6.8, 8.3, 8.7, 9.5, 12.2, 13.3, 15.0, 15.7, 17.5, 18.7, 19.5, 20.2, 21.4, 24.7, and 26.2 ±0.2 degrees two-theta. The most characteristic peak of Form G is at about 6.8 ±0.2 degrees two-theta. The diffraction pattern is reproduced in Fig. 10.

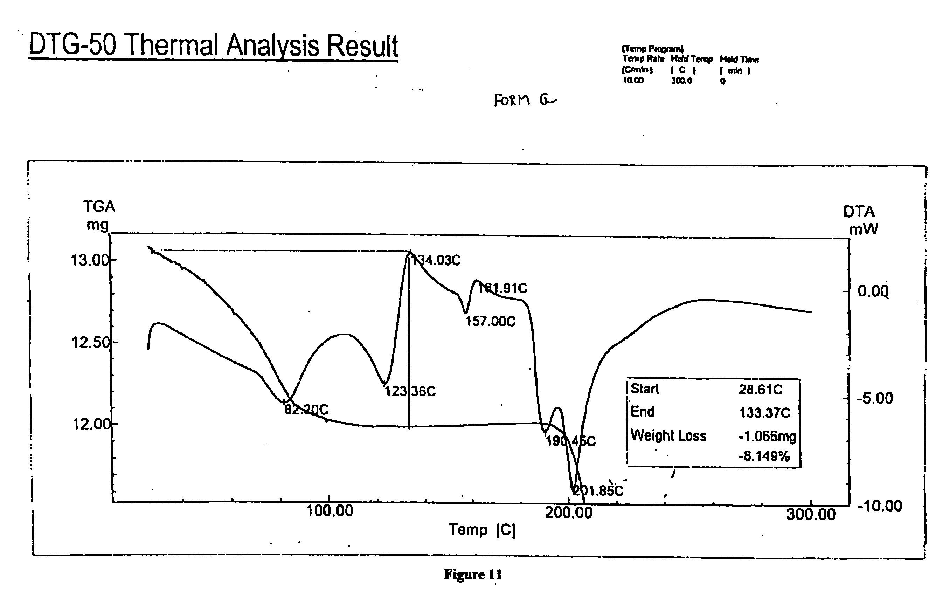

The DTG thermal profile of Form G is shown in Fig.11. The DSC thermal profile of Form G contains two desorption endotherm with a peak maxima at about 82 and 123°C, a subsequent recrystallization exotherm around 134°C, and a double endotherm of melting and decomposition at about 190°C and about 202°C.

The solvation states of Form G is characterized by TGA and Karl Fisher analysis. Form G has a weight loss of about 8% which occurs in the TGA mainly at about 80°C. Karl Fischer analysis of Form G reveals minimal quantities of water (below 1%). Thus, the TGA and Karl Fisher analysis indicate that Form G is a solvate form of zolpidem hemitartrate.

### Zolpidem hemitartrate Form H

Zolpidem hemitartrate Form H ("Form H") is a mixed solvate and hydrate crystalline form of zolpidem hemitartrate.

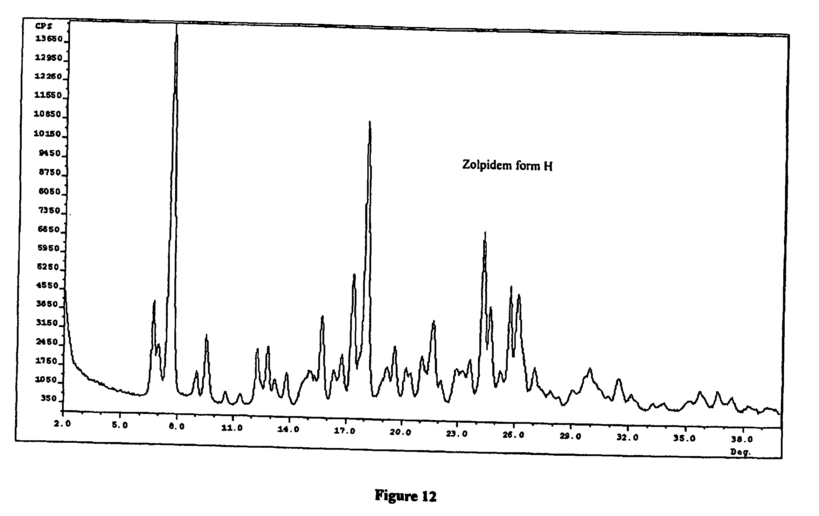

Zolpidem hemitartrate Form H is characterized by an X-ray diffraction pattern with peaks at about 6.7, 7.7, 9.0, 9.5, 12.2, 13.2, 13.9, 15.7, 16.8, 17.4, 18.0, 19.6, 21.7, 24.3, 24.7, 25.7, and 26.2 ± 0.2 degrees two-theta. The most characteristic peaks of Form H is at about 7.7, 17.4, 18.0 and 24.3 ±0.2 degrees two-theta. The diffraction pattern is reproduced in Fig. 12.

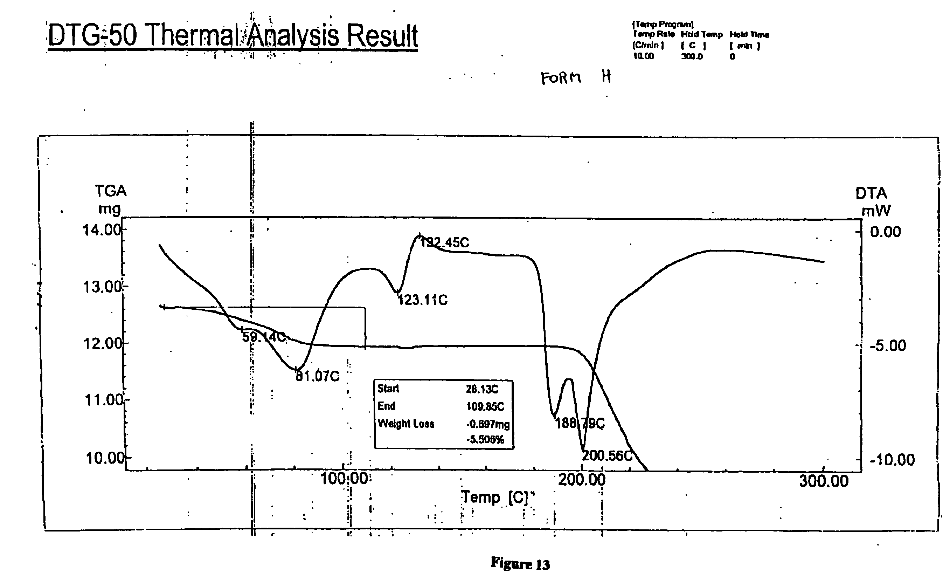

The DTG thermal profile of Form H is shown in Fig. 13. The DSC thermal profile of Form H contains an endotherm of desorption at about 81 °C followed by a recrystallization endotherm at about 132 °C. The thermal profile further shows a double endotherm of melting and decomposition at 189 °C and 200 °C.

The solvation states of Form H is characterized by TGA and Karl Fisher analysis. TGA analysis indicates that Form H has a weight loss of about 5.5 % mainly at about 80°C. Karl Fischer analysis of Form H reveals about 0.7 to about 3.2 % water. Thus, the TGA and Karl Fisher analysis indicate that Form H is a mixed solvate and hydrate form of zolpidem hemitartrate.

### Zolpidem hemitartrate Form L

Zolpidem hemitartrate Form L ("Form L") is a dihydrate crystal form of zolpidem hemitartrate.

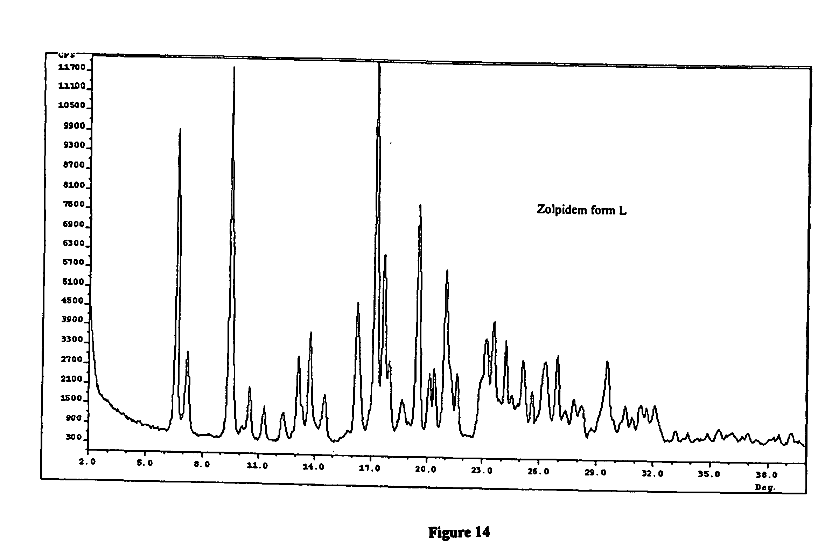

Zolpidem hemitartrate Form L is characterized by an X-ray diffraction pattern with peaks at about 6.8, 7.5, 9.7, 10.6, 13.2, 13.9, 16.4, 17.3, 17.7, 19.6, 21.1, 21.6, 23.2, 23.6, 26.3, 27.1 and 29.7 ± 0.2 degrees two-theta. The most characteristic peaks of Form L are at about 6.8, 9.7, 17.3, 19.6 and 21.1 ±0.2 degrees two-theta. The diffraction pattern is reproduced in Fig. 14.

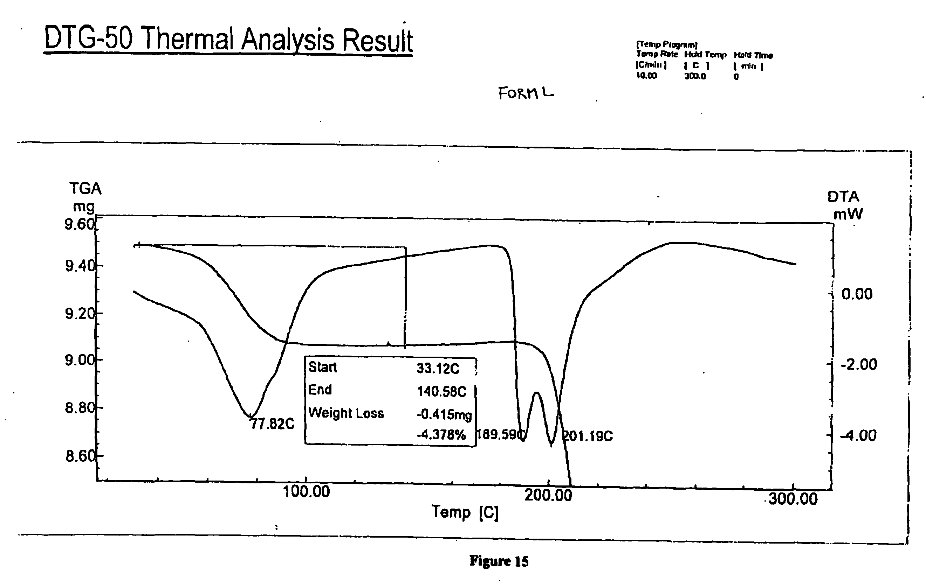

The DTG thermal profile of Form L is shown in Fig. 15. The DSC thermal profile of Form L contains an endotherm of desorption at about 78 °C. The DSC thermal profile further shows a double endotherm of melting and decomposition at 190 °C and 201 °C.

The hydration state of Form L is unequivocably by TGA and Karl Fisher analysis. Form L has a weight loss of about 4.4 % mainly at 80°C which corresponds to about 2 water molecules per molecule of zolpidem hemitartrate. Karl Fischer analysis of Form L reveals about 4.3 % water. Thus, the TGA and Karl Fisher analysis indicate that Form L is a dihydrate of zolpidem hemitartrate.

### Procedures for Crystallizing Polymorphs of Zolpidem Hemitartrate

### General Description

The novel forms of zolpidem hemitartrate disclosed herein are optionally formed by: (1) exposing various solid forms of zolpidem hemitartrate to water vapor or solvent vapors; (2) suspending the crystals as a slurry of zolpidem hemitartrate particles in a solvent; (3) granulation; (4) crystallization; or (5) heat treatment. It will be understood by those of skill in the art that other methods may also be used to form the polymorphs disclosed herein.

### Formation of Polymorphs by Vapor Exposure

Examples of procedures for exposing powder to solvent vapors in a are provided in Examples 6 to 16. Optionally vapor treatment may be performed by placing, about 0.1 g to about 0.2 g of a solid form of zolpidem hemitartrate in a small open container. The container can be a flat 5 cm (or less) diameter dish. The container can be optionally a bottle of a volume of about 10 ml. The open bottle is optionally introduced into a chamber of a volume from about 50 ml to about 150 ml. The chamber may be a bottle. The chamber preferably contains about 5 to about 30 ml of a solvent. The chamber is sealed creating a solvent saturated atmosphere. Preferably, the sample is then stored for a time period ranging from about 5 to about 10 days. Most preferably the sample is stored for about 7 days. When the solvent is water the degree of chamber humidity may be regulated using salts or salt solutions such as potassium sulphate, zinc nitrate, potassium acetate, ammonium sulphate, and the chamber is a 20x20x10 cm size sealed chamber apposite for this purpose (hygroscopicity chamber). The solid zolpidem hemitartrate is then analyzed.

### Formation of Polymorphs by Slurry

Examples of procedures for slurry are provided in Examples 17 to 25. Forming the suspension optionally includes mixing solid zolpidem hemitartrate with a solvent in which complete dissolution does not occur. The mixture is optionally stirred for a period of time needed to achieve the desired transformation, and the solid compound collected and analyzed. *Formation of Polymorphs by Granulation*

Examples of a procedure for granulating are provided in Examples 26 to 31. Granulation optionally includes mixing solid zolpidem hemitartrate with a minimal amount of solvent insufficient to dissolve the material, and stirring the mixture at room temperature for the time needed to cause the desired transformation. The mixture is optionally stirred for a period of time and the compound collected and analyzed.

### Formation of Polymorphs by Heating

Examples of procedures for performing crystal structure transformations by heating are provided in Examples 4 and 5. The sample to be heated can be in small quantities (about 0.1 to about 0.2 g) or larger quantities (kilograms or more). As the quantity of material to be heated increases, the time needed to cause a physical transformation will increase from several minutes to several hours or adversely the temperature needed to cause the transformation will increase. It should be noted that high temperatures employed to cause phase transformations may cause unwanted chemical reactions and decomposition.

### SMALL PARTICLES OF ZOLPIDEM HEMITARTRATE

The present invention also provides zolpidem hemitartrate having a relatively small particle size and a corresponding relatively large surface area.

It has long been recognized that when a pharmaceutical composition containing a drug which is orally administered to subjects, a dissolution step is essential for the drug to be absorbed through gastrointestinal tract. A drug may have insufficient bioavailability because of the poor solubility in the gastrointestinal tract, consequently the drug passes through the site of absorption before it completely dissolves in the fluids.

Bioavailability, particularly of slightly soluble active compounds is highly dependent on the surface area of the particles and the surface area is inversely related to the size of the compound. Thus particles having relatively small particle size have a relatively greater surface area and an increased solubility rate in gastrointestinal tract.

Small zolpidem hemitartrate particles can be achieved using methods well known in the art. (See US Patents Nos. 4,151,273; 4,196,188; 4,302,446; 4,332, 721; 4,840,799; and 5,271,944, incorporated herein by reference.) Micronization as provided in Example ? in one method of generating small zolpidem hemitartrate particles. Particle size was measured by a laser diffraction instrument (Malvem Mastersizer S). The sample was analysed after proper dispersion in a solution of dioctylsulfosuccinate sodium salt in hexane (0.02% w/w).

In one embodiment, the invention provides zolpidem hemitartrate in which substantially all zolpidem hemitartrate particles have a particle size of up to about 200 micrometer. It will be understood by those of skill in the art that this embodiment includes pharmaceutical compositions containing a therapeutically effective amount of zolpidem hemitartrate.

According to another embodiment, the present invention provides zolpidem hemitartrate particles in which substantially all zolpidem hemitartrate particles, have a particle size of up to about 50 microns. It will be understood by those of skill in the art that this embodiment includes pharmaceutical compositions containing a therapeutically effective amount of zolpidem hemitartrate.

### A PHARMACEUTICAL COMPOSITION CONTAINING ZOLPIDEM HEMITARTRATE

According to another aspect, the present invention relates to a pharmaceutical composition comprising one or more of the novel crystal forms of zolpidem hemitartrate disclosed herein and at least one pharmaceutically acceptable excipient. Such pharmaceutical compositions may be administered to a mammalian patient in a dosage form.

The dosage forms may contain one or more of the novel forms of zolpidem hemitartrate or, alternatively, may contain one or more of the novel forms of zolpidem hemitartrate as part of a composition. Whether administered in pure form or in a composition, the zolpidem hemitartrate form(s) may be in the form of a powder, granules, aggregates or any other solid form. The compositions of the present invention include compositions for tableting. Tableting compositions may have few or many components depending upon the tableting method used, the release rate desired and other factors. For example, compositions of the present invention may contain diluents such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents such calcium carbonate and calcium diphosphate and other diluents known to one of ordinary skill in the art. Yet other suitable diluents include waxes, sugars (*e*.*g*. lactose) and sugar alcohols like mannitol and sorbitol, acrylate polymers and copolymers, as well as pectin, dextrin and gelatin.

Other excipients contemplated by the present invention include binders, such as acacia gum, pregelatinized starch, sodium alginate, glucose and other binders used in wet and dry granulation and direct compression tableting processes; disintegrants such as sodium starch glycolate, crospovidone, low-substituted hydroxypropyl cellulose and others; lubricants like magnesium and calcium stearate and sodium stearyl fumarate; flavorings; sweeteners; preservatives; pharmaceutically acceptable dyes and glidants such as silicon dioxide.

Dosage forms may be adapted for administration to the patient by oral, buccal, parenteral, ophthalmic, rectal and transdermal routes. Oral dosage forms include tablets, pills, capsules, troches, sachets, suspensions, powders, lozenges, elixirs and the like. The novel forms of zolpidem hemitartrate disclosed herein also may be administered as suppositories, ophthalmic ointments and suspensions, and parenteral suspensions, which are administered by other routes. The most preferred route of administration of the zolpidem hemitartrate forms of the present invention is oral.

Capsule dosages will contain the solid composition within a capsule which may be coated with gelatin. Tablets and powders may also be coated with an enteric coating. The enteric-coated powder forms may have coatings comprising phthalic acid cellulose acetate, hydroxypropylmethyl cellulose phthalate, polyvinyl alcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric-coating.

The currently marketed form of zolpidem hemitartrate (AMBIEN) are a 5 and a 10 mg tablet which includes the following inactive ingredients: hydroxypropyl methylcellulose, lactose, magnesium stearate, microcrystalline cellulose, polyethylene glycol, sodium starch glycolate, titanium dioxide; the 5 mg tablet also contains FD&C Red No. 40, iron oxide colorant, and polysorbate 80.

The function and advantage of these and other embodiments of the present invention will be more fully understood from the examples below. The following examples are intended to illustrate the benefits of the present invention, but do not exemplify the full scope of the invention.

### EXAMPLES

### EXAMPLE 1:

### Synthesis of zolpidem base.

5 g (17.7 mmol) ofZolpidic acid is suspended in 50 ml of toluene and 0.15 ml of dimethylformamide. This mixture is cooled to 15-28 °C. 1.7 ml (23.3 mmol) of thionyl chloride is added into the mixture at this temperature for 1 h, then it is stirred for 4 hrs at 35-40 °C.

After formation of acid chloride the thionyl chloride excess is removed by distillation. The volume of the reaction mixture is adjusted to 50 ml by toluene, then it is cooled to -5-0 °C, then dimethylamine gas is introduced into the reaction mixture until the pH is 8.5-9.5. Precipitation ofZolpidem base starts almost immediately.

The suspension is cooled to -10-(-12) °C and mixed for 1 h. The crude product is filtered and washed consecutively with toluene, 5 % cooled water solution of NH4CO3and cooled water. The product is dried under vacuum 4.1 g (assay 97.6 % by HPLC, yield 80 %) Zolpidem base is obtained.

### EXAMPLE 2

### Synthesis of zolpidem base.

5 g (17.7 mmol) ofZolpidic acid is suspended in 50 ml of toluene and 0.3 ml of dried dimethylformamide. This mixture is cooled to 15-28 °C. 1.4 ml (19.5 mmol) of thionyl chloride is added into the mixture at this temperature for 1 h, then it is stirred for 4 hrs at 20-25 °C.

After formation of acid chloride the thionyl chloride excess is removed by distillation. The volume of the reaction mixture is adjusted to 50 ml by toluene, then it is cooled to -5-0 °C, then dimethylamine gas is introduced into the reaction mixture until the pH is 8.5-9.5. Precipitation of Zolpidem base starts almost immediately.

The suspension is cooled to 0-5 °C and mixed for 1 h. The crude product is filtered and washed consecutively with toluene, 5 % cooled water solution of NH4CO3and cooled water. The product is dried under vacuum .4.4 g (assay 94.6 % by HPLC, yield 70.7 %) Zolpidem base is obtained.

### EXAMPLE 3

### Preparation of zolpidem hemitartrate Form A by crystallization.

Crude zolpidem hemitartrate (6.1 g) is suspended in 90 ml of methanol and the mixture solution is heated to 44-46°C. The solution is agitated at this temperature for 30 minutes. The 6.1 g crude salt is dissolved after 30 minutes agitating at this temperature. The clear mixture solution is cooled to room temperature and stirred for 3 hours. The methanol is evaporated in vacuum to a mixture solution volume of 12 ml. The resulting mixture solution is cooled and kept for 12 hours at 0-5°C, and then filtered. The crystalline product is dried under vacuum (70 to 100 mbar) at 38°C for 12 hours.

### EXAMPLE 4

### Preparation of zolpidem hemitartrate Form C by the heating zolpidem hemitartrate

A solid form of zolpidem hemitartrate (100 mg) was heated at 130°C for 1/2 hour to yeld anhydrous Form C by placing the sample in an oven inside an open container.

### EXAMPLE 5

### Preparation of zolpidem hemitartrate Form C by the heating zolpidem hemitartrate

A solid form of zolpidem hemitartrate (100 mg) was heated at 160°C for 1/4 hour to yeld anhydrous Form C by placing the sample in an oven inside an open container.

### EXAMPLE 6

### Formation of zolpidem hemitartrate Form D by water vapor absorption of Form A.

A sample of zolpidem hemitartrate Form A (100 mg) was stored in a flat 4 cm diameter dish. The dish was introduced into a 100 ml chamber of 80 % relative humidity for a period of 1 week, at ambient temperature. The resulting solid was Zolpidem hemitartrate form D.

### EXAMPLE 7

### Formation of zolpidem hemitartrate Form D by water vapor absorption of Form C.

A sample of zolpidem hemitartrate Form C (100 mg) was stored in a flat 4 diameter dish. The dish was introduced into a 100 ml chamber of 100% relative humidity for a period of 1 week, at ambient temperature. The resulting solid was Zolpidem hemitartrate form D.

### EXAMPLE 8

### Formation of zolpidem hemitartrate Form E by water vapor absorption of Form D.

A sample of zolpidem hemitartrate Form D (100 mg) was stored in a flat 4 diameter dish. The dish was introduced into a 100 ml chamber of 100% relative humidity for a period of 1 week, at ambient temperature. The resulting solid was Zolpidem hemitartrate form E.

### EXAMPLE 9

### Formation of zolpidem hemitartrate Form E by water vapor absorption of Form A.

A sample of zolpidem hemitartrate Form A (100 mg) was stored in a flat 4 diameter dish. The dish was introduced into a 100 ml chamber of 100% relative humidity for a period of 1 week, at ambient temperature. The resulting solid was Zolpidem hemitartrate form E.

### EXAMPLE 10

### Formation of zolpidem hemitartrate Form F by methanol vapor absorption of

### Form A.

A sample of zolpidem hemitartrate Form A (100 mg) was stored in a 10 ml bottle. The bottle was introduced into a 100 ml chamber containing 30 ml of methanol. The chamber was sealed creating an atmosphere of saturated methanol vapor. Zolpidem hemitartrate Form F was obtained after the sample was exposed to methanol vapors for a period of 1 week, at ambient temperature.

### EXAMPLE 11

### Formation of zolpidem hemitartrate Form F by methanol vapor absorption of

### Form C.

A sample of zolpidem hemitartrate Form C (100 mg) was stored in a 10 ml bottle. The bottle was introduced into a 100 ml chamber containing 20 ml of a methanol. The chamber was sealed creating an atmosphere of saturated methanol vapor. Zolpidem hemitartrate Form F was obtained after the sample was exposed to methanol vapors for a period of 1 week, at ambient temperature.

### EXAMPLE 12

### Formation of zolpidem hemitartrate Form D by ethanol vapor absorption of

### Form A.

A sample of zolpidem hemitartrate Form A (100 mg) was stored in a 10 ml bottle. The bottle was introduced into a 100 ml chamber containing 20 ml of a ethanol. The chamber was sealed creating an atmosphere of saturated ethanol vapor. Zolpidem hemitartrate Form D was obtained after the sample was exposed to ethanol vapors for a period of 1 week, at ambient temperature.

### EXAMPLE 13

### Formation of zolpidem hemitartrate Form D by ethanol vapor absorption of

### Form C.

A sample of zolpidem hemitartrate Form C (100 mg) was stored in a 10 ml bottle. The bottle was introduced into a 100 ml chamber containing 20 ml of a ethanol. The chamber was sealed creating an atmosphere of saturated ethanol vapor. Zolpidem hemitartrate Form D was obtained after the sample was exposed to ethanol vapors for a period of 1 week, at ambient temperature.

### EXAMPLE 14

### Formation of zoloidem hemitartrate Form C by exposure of Form A to isopropanol vapors.

A sample of zolpidem hemitartrate Form C (100 mg) was stored in a 10 ml bottle. The bottle was introduced into a 100 ml chamber containing 20 ml of isopropanol. The chamber was sealed creating an atmosphere of saturated isopropanol vapor. Zolpidem hemitartrate Form C was obtained after the sample was exposed to isopropanol vapors for a period of 1 week, at ambient temperature.

### EXAMPLE 15

### Formation of zolpidem hemitartrate Form C by exposure of Form A to butanol vapors.

A sample of zolpidem hemitartrate Form A (100 mg) was stored in a 10 ml bottle. The bottle was introduced into a 100 ml chamber containing 20 ml of butanol. The chamber was sealed creating an atmosphere of saturated butanol vapor. Zolpidem hemitartrate Form C was obtained after the sample was exposed to butanol vapors for a period of 1 week, at ambient temperature.

### EXAMPLE 16

### Formation of zolpidem hemitartrate Form G by exposure of Form A to ethyl acetate vapors.

A sample of zolpidem hemitartrate Form A (100 mg) was stored in a 10 ml bottle. The bottle was introduced into a 100 ml chamber containing 20 ml of ethyl acetate. The chamber was sealed creating an atmosphere of saturated ethyl acetate vapor. Zolpidem hemitartrate Form G was obtained after the sample was exposed to ethyl acetate vapors for a period of 1 week, at ambient temperature.

### EXAMPLE 17

### Formation of zolpidem hemitartrate Form C by forming a slurry of Form A in isopropanol.

A sample of zolpidem hemitartrate Form A (2.2 g) was suspended in 11.0 ml of isopropanol. The slurry was stirred for 24 hours. The resulting solid was filtered and analyzed by XRD. The XRD showed the product to be zolpidem hemitartrate Form C.

### EXAMPLE 18

### Formation of zolpidem hemitartrate Form D by forming a slurry of Form A in acetone.

A sample of zolpidem hemitartrate Form A (2.2 g) was suspended in 11.0 ml of acetone. The slurry was stirred for 24 hours. The resulting solid was filtered and analyzed by XRD. The XRD showed the product to be zolpidem hemitartrate Form D.

### EXAMPLE 19

### Formation of zolpidem hemitartrate Form D by forming a slurry of Form A in ethyl acetate.

A sample of zolpidem hemitartrate Form A (2.2 g) was suspended in 5.0 ml of ethyl acetate. The slurry was stirred for 24 hours. The resulting solid was filtered and analyzed by XRD. The XRD showed the product to be zolpidem hemitartrate Form D.

### EXAMPLE 20

### Formation of zolpidem hemitartrate Form E by forming a slurry of Form A in water.

A sample of zolpidem hemitartrate Form A (2.5 g) was suspended in 17.0 ml of water. The slurry was stirred for 24 hours. The resulting solid was filtered and analyzed by XRD. The XRD showed the product to be zolpidem hemitartrate Form E.

### EXAMPLE 21

### Formation of zolpidem hemitartrate Form E by forming a slurry of Form C in water.

A sample of zolpidem hemitartrate Form C (2.6 g) was suspended in 17.0 ml of water. The slurry was stirred for 24 hours. The resulting solid was filtered and analyzed by XRD. The XRD showed the product to be zolpidem hemitartrate Form E.

### EXAMPLE 22

### Formation of Zolpidem Hemitartrate Form G by forming a slurry of Form C in methanol.

A sample of zolpidem hemitartrate Form C (2.5 g) was suspended in 4.35 ml of methanol. The slurry was stirred for 24 hours. The resulting solid was filtered and analyzed by XRD. The XRD showed the product to be zolpidem hemitartrate Form G.

### EXAMPLE 23

### Formation of zolpidem hemitartrate Form G by forming a slurry of Form C in ethanol.

A sample of zolpidem hemitartrate Form C (2.5 g) was suspended in 4.0 ml of ethanol. The slurry was stirred for 24 hours. The resulting solid was filtered and analyzed by XRD. The XRD showed the product to be zolpidem hemitartrate Form G.

### EXAMPLE 24

### Formation of zolpidem hemitartrate Form H by forming a slurry of Form A in ethanol.

A sample of zolpidem hemitartrate Form A (2.5 g) was suspended in 3.5 ml of ethanol. The slurry was stirred for 24 hours. The resulting solid was filtered and analyzed by XRD. The XRD showed the product to be zolpidem hemitartrate Form H.

### EXAMPLE 25

### Formation of zolpidem hemitartrate Form H by forming a slurry of Form A in methanol.

A sample ofzolpidem hemitartrate Form A (2.5 g) was suspended in 4.35 ml of methanol. The slurry was stirred for 24 hours. The resulting solid was filtered and analyzed by XRD. The XRD showed the product to be zolpidem hemitartrate Form H.

### EXAMPLE 26

### Formation of zolpidem hemitartrate Form D by granulating Form A in isopropanol.

A sample of zolpidem hemitartrate Form A (3.3 g) was suspended in 2.6 ml of isopropanol. The wet powder was stirred for 24 hours. The resulting solid was filtered and analyzed by XRD. The XRD showed the product to be zolpidem hemitartrate Form

### D. EXAMPLE 27

### Formation of zolpidem hemitartrate Form D by granulating Form A in butanol.

A sample of zolpidem hemitartrate Form A (1.6 g) was suspended in 1.1 ml of butanol. The wet powder was stirred for 24 hours. The resulting solid was filtered and analyzed by XRD. The XRD showed the product to be zolpidem hemitartrate Form D.

### EXAMPLE 28

### Formation of zolpidem hemitartrate Form G by granulating Form C in ethanol.

A sample of zolpidem hemitartrate Form C (2.5 g) was suspended in 1.2 ml of ethanol. The wet powder was stirred for 24 hours. The resulting solid was filtered and analyzed by XRD. The XRD showed the product to be zolpidem hemitartrate Form G.

### EXAMPLE 29

### Formation of zolpidem hemitartrate Form G by granulating Form C in methanol.

A sample of zolpidem hemitartrate Form C (2.5 g) was suspended in 1.1 ml of methanol. The wet powder was stirred for 24 hours. The resulting solid was filtered and analyzed by XRD. The XRD showed the product to be zolpidem hemitartrate Form G.

### EXAMPLE 30

### Formation of zolpidem hemitartrate Form H by granulating Form A in ethanol.

A sample of zolpidem hemitartrate Form A (2.2 g) was suspended in 1.1 ml of ethanol. The wet powder was stirred for 24 hours. The resulting solid was filtered and analyzed by XRD. The XRD showed the product to be zolpidem hemitartrate Form H.

### EXAMPLE 31

### Formation of zolpidem hemitartrate Form H by granulating Form A in methanol.

A sample of zolpidem hemitartrate Form A (3.0 g) was suspended in 1.3 ml of methanol. The wet powder was stirred for 24 hours. The resulting solid was filtered and analyzed by XRD. The XRD showed the product to be zolpidem hemitartrate Form H.

### EXAMPLE 32

### Formation of zolpidem hemitartrate Form L by Crystallization.

Zolpidem hemitartrate (5 g) was dissolved in a mixture of 43.6 ml of methanol and 3.4 ml water (methanol:water ratio is 13:1) at 60°C. The solution was filtered and cooled to room temperature. Upon reaching 30°C precipitation of Zolpidem hemitartrate started. The suspension was mixed at room temperature for 3 hrs then methanol was evaporated by vacuum distillation. The suspension was stored for 12 hrs at 0-5°C. The sample was filtered and dried in vacuum (150 mbar) at 40°C for 16 hrs. The XRD analysis showed the product to be a novel zolpidem hemitartrate designated Form L.

### EXAMPLE 33

### Micronization of zolpidem hemitartrate

Pure dry zolpidem hemitartrate was micronized in an air jet micronizer (CHRISPRO Jetmill MC-200KX, BD). The feeding rate was set at 9.0 kg/hr. The feeding air pressure was set at 6.0 bar. The grinding air pressure was set 3.5 bar. The particle size of the micronized zolpidem hemitartrate was found to be less than 20 microns Malvem laser diffraction Mastersizer S.

### EXAMPLE 34

### X-Ray powder diffraction spectra of micronization of zolpidem hemitartrate Form A

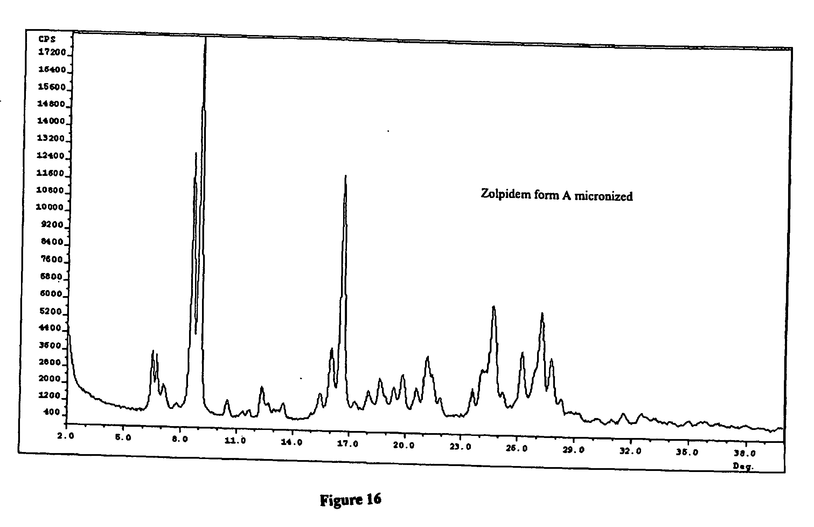

Zolpidem hemitartrate Form A was micronized as in Example 33 to a particle size up to 20 microns as determined by laser diffraction. The X-Ray powder diffraction spectra showed an unexpected peak at about 8.6 degrees two-theta. Other unexpected peaks were observed at 6.7, 11.2, 15.4 and 17.3 ±0.2 degrees two-theta. An X-Ray diffraction pattern for micronized Form A is shown in Fig. 16.

### EXAMPLE 35

### Zolpidem hemitartrate Form B

Zolpidem hemitartrate Form B may be prepared by dissolving any solid form of zolpidem hemitartrate in methanol to form a solution; concentrating the solution by evaporation of methanol in a vacuum; crystallizing the zolpidem hemitartrate Form A from the solution; and, heating zolpidem hemitartrate Form A to about 130°C for about 30 minutes.

Zolpidem hemitartrate Form B is characterized by a powder X-ray diffraction pattern at about 8.2, 17.3, and 18.4 ±0. 2 degrees two-theta

Further particular and preferred aspects of the invention are set out in the accompanying independent and dependent clauses. Features of the dependent clauses may be combined with those of the independent clauses and/or independent claims as appropriate and in combinations other than those explicitly set out in the clauses and clauses.
1. A Zolpidem hemitartrate hydrate.
2. A Zolpidem hemitartrate monohydrate.
3. A Zolpidem hemitartrate dihydrate.
4. A Zolpidem hemitartrate trihydrate.
5. A Zolpidem hemitartrate tetrahydrate.
6. A Zolpidem hemitartrate solvate.
7. The zolpidem hemitartrate solvate of clause 6, selected from the group consisting of zolpidem hemitartrate isopropanol, zolpidem hemitartrate butanol, zolpidem hemitartrate ethylacetate and zolpidem hemitartrate acetone.
8. Zolpidem hemitartrate anhydrous.
9. Zolpidem hemitartrate with not more than 1% water content.
10. An anhydrous Zolpidem hemitartrate Form C.
11. The Zolpidem hemitartrate of clause 10, with water content of not more than 1 %.
12. Zolpidem hemitartrate Form C, characterized by an X-ray powder diffraction pattern having peaks at about 7.3, 9.5, 17.8 and 23.8 ± 0.2 degrees two-theta.
13. The zolpidem hemitartrate of clause 12, further characterized by an X-ray powder diffraction pattern having peaks at about 10.7, 12.4, 13.0, 13.8, 14.6, 16.2, 18.9, 19.5, 20.3, 21.3, 23.5, 25.0, and 27.0 ± 0.2 degrees two-theta.
14. The zolpidem hemitartrate of clause 12, having particles up to about 200 microns in size, as measured by laser diffraction.
15. The zolpidem hemitartrate of clause 12, having particles up to about 50 microns in size as measured by laser diffraction.
16. A pharmaceutical composition comprising a therapeutically effective amount of the zolpidem hemitartrate of clause 12, and a pharmaceutically acceptable carrier.
17. A method for treating a patient suffering from insomnia by administering a therapeutically effective amount of the zolpidem hemitartrate Form C.
18. Zolpidem hemitartrate Form D monohydrate.
19. Zolpidem hemitartrate Form D, characterized by a water content of about 2.3 % to about 2.7 % by weight.
20. Zolpidem hemitartrate Form D hemiethanolate.
21. Zolpidem hemitartrate Form D, characterized by an X-ray powder diffraction pattern having peaks at about 7.1, 9.5, 14.1, 19.6 and 24.5 ±0.2 degrees two-theta.
22. The zolpidem hemitartrate of clause 21, further characterized by an X-ray powder diffraction pattern having peaks at about 8.4, 10.2, 12.2, 12.9, 13.2, 15.9, 16.3, 17.7, 18.8, 21.0, 21.7, 23.0, 23.6, 25.9, 26.5, 30.0, and 30.6 ± 0.2 degrees two-theta.
23. The zolpidem hemitartrate of clause 21, having particles up to about 200 microns in size, as measured by laser diffraction.
24. The zolpidem hemitartrate of clause 21, having particles up to about 50 microns in size, as measured by laser diffraction.
25. A pharmaceutical composition comprising a therapeutically effective amount of the zolpidem hemitartrate of clause 21, and a pharmaceutically acceptable carrier.
26. A method for treating a patient suffering from insomnia by administering a therapeutically effective amount of zolpidem hemitartrate Form D.
27. Zolpidem hemitartrate Form E dihydrate.
28. Zolpidem hemitartrate Form E trihydrate.
29. Zolpidem hemitartrate Form E tetrahydrate.
30. Zolpidem hemitartrate Form E, characterized by a water content from about 5.0% to about 8.5% by weight.
31. Zolpidem hemitartrate Form E, characterized by an X-ray powder diffraction pattern having peaks at about 5.2, 7.9, 10.4, 17.2, 18.0 and 18.8 ±0.2 degrees two-theta.
32. The zolpidem hemitartrate of clause 31, further characterized by an X-ray powder diffraction pattern having peaks at about 6.8, 11.0, 13.7, 14.2, 15.8, 16.1, 19.7, 20.1, 22.2, 24.4, 25.2, 25.9, 28.5, 31.0, 31.8 and 32.5 ±0.2 degrees two-theta.
33. The zolpidem hemitartrate of clause 31, having particles up to about 200 microns in size, as measured by laser diffraction.
34. The zolpidem hemitartrate of clause 31, having particles up to about 50 microns in size, as measured by laser diffraction.
35. A pharmaceutical composition comprising a therapeutically effective amount of the zolpidem hemitartrate of clause 31 and a pharmaceutically acceptable carrier.
36. A method for treating a patient suffering from insomnia by administering a therapeutically effective amount of the zolpidem hemitartrate Form E.
37. Zolpidem Form F methanolate.
38. The zolpidem hemitartrate Form F, characterized by a methanol content of about 5.5% by weight.
39. Zolpidem hemitartrate Form F, characterized by an X-ray powder diffraction pattern having peaks at about 7.6 and 18.0 ± 0.2 degrees two-theta.
40. The zolpidem hemitartrate of clause 39, further characterized by an X-ray powder diffraction pattern having peaks at about 9.0, 12.2,12.7,15.7,16.7,17.3,19.6, 21.6, 24.3, 24.7, 25.7, and 26.1 ±0.2 degrees two-theta.
41. The zolpidem hemitartrate of clause 39, having particles up to about 200 microns in size, as measured by laser diffraction.
42. The zolpidem hemitartrate of clause 39, having particles up to about 50 microns in size, as measured by laser diffraction.
43. A pharmaceutical composition comprising a therapeutically effective amount of the zolpidem hemitartrate of clause 39, and a pharmaceutically acceptable carrier.
44. A method for treating a patient suffering from insomnia by administering a therapeutically effective amount of the zolpidem hemitartrate Form F.
45. Zolpidem hemitartrate Form G solvate.
46. Zolpidem hemitartrate Form G, characterized by an X-ray powder diffraction pattern having peaks at about 6.8 ±0.2 degrees two-theta.
47. The zolpidem hemitartrate of clause 46, further characterized by an X-ray powder diffraction pattern having peaks at about 8.3, 8.7, 9.5, 12.2, 13.3, 15.0, 15.7, 17.5, 18.7, 19.5, 20.2, 21.4, 24.7, and 26.2 ±0.2 ± 0.2 degrees two-theta.
48. The zolpidem hemitartrate of clause 46, having particles up to about 200 microns in size, as measured by laser diffraction.
49. The zolpidem hemitartrate of clause 46, having particles up to about 50 microns in size, as measured by laser diffraction.
50. A pharmaceutical composition comprising a therapeutically effective amount of the zolpidem hemitartrate clause 46, and a pharmaceutically acceptable carrier.
51. A method for treating a patient suffering from insomnia by administering a therapeutically effective amount of the zolpidem hemitartrate Form G.
52. Zolpidem hemitartrate Form H, characterized by an X-ray powder diffraction pattern having peaks at about 7.7, 17.4, 18.0 and 24.3 ±0.2 degrees two-theta.
53. The zolpidem hemitartrate of clause 52, further characterized by an X-ray powder diffraction pattern having peaks at about 6.7, 7.7, 9.0, 9.5, 12.2, 13.2, 13.9, 15.7, 16.8, 19.6, 21.7, 24.7, 25.7, and 26.2 ± 0.2 degrees two-theta.
54. The zolpidem hemitartrate of clause 52, having particles up to about 200 microns in size, as measured by laser diffraction.
55. The zolpidem hemitartrate of clause 52, having particles up to about 50 microns in size, as measured by laser diffraction.
56. A pharmaceutical composition comprising a therapeutically effective amount of the zolpidem hemitartrate of clause 52, and a pharmaceutically acceptable carrier.
57. A method for treating a patient suffering from insomnia by administering a therapeutically effective amount of the zolpidem hemitartrate Form H.
58. Zolpidem hemitartrate form L dihydrate.
59. Zolpidem hemitartrate Form L, characterized by a water content of about 4.3% by weight.
60. Zolpidem hemitartrate Form L, characterized by an X-ray powder diffraction pattern having peaks at about 6.8, 9.7, 17.3, 19.6 and 21.1 ±0.2 degrees two-theta.
61. The zolpidem hemitartrate of clause 60, further characterized by an X-ray powder diffraction pattern having peaks at about 7.5, 10.6, 13.2, 13.9, 16.4, 17.7, 21.6, 23.2, 23.6, 26.3, 27.1 and 29.7 ± 0.2 degrees two-theta.
62. The zolpidem hemitartrate of clause 60, having particles up to about 200 microns in size, as measured by laser diffraction.
63. The zolpidem hemitartrate of clause 60, having particles up to about 50 microns in size, as measured by laser diffraction.
64. A pharmaceutical composition comprising a therapeutically effective amount of the zolpidem hemitartrate of clause 60, and a pharmaceutically acceptable carrier.
65. A method for treating a patient suffering from insomnia by administering a therapeutically effective amount of the zolpidem hemitartrate Form L.
66. A method for synthesizing zolpidem hemitartrate, comprising the steps of:
   (a) forming a zolpidic acid halide from the zolpidic acid;
   (b) reacting zolpidem acid halide, with dimethyl amine, to form zolpidem base;
   (c) forming zolpidem hemitartrate salt from the zolpidem base.
67. The method of clause 66, wherein the step of forming a zolpidic acid halide further comprises using DMF as a co-solvent for the reaction to facilitate the contact of thionyl chloride and zolpidic acid.
68. The method of clause 66, further comprising using toluene as a crystallization solvent to purifies effectively zolpidem.
69. The method of clause 66, further comprising using DMF as co-solvent to improve the purification effect of Zolpidem.
70. The method of clause 66, further comprising using toluene as a transport medium for the effective removal of the excess of thionyl chloride from the reaction mass.
71. The method of clause 66, wherein the step of forming a zolpidic acid halide further comprises, using toluene as a reaction medium in which the acid chloride precipitates avoiding the undesired additional chlorination reaction of zolpidic acid.
72. The method of clause 66, wherein the step of forming a zolpidic acid halide further comprises, using toluene as a crystallization solvent for zolpidem and acid chloride.
73. The method of clause 66, wherein the step of forming a zolpidic acid halide further comprises, using toluene as a reaction medium for "one pot" reaction from zolpidic acid to zolpidem.
74. The method of clause 66, wherein the halide is chloride.
75. The method of clause 74, wherein the step of forming the acid chloride is performed using thionyl chloride.
76. The method of clause 74, wherein the step of forming the acid halide is performed using toluene as a solvent.
77. The method of clause 66, further comprising the step of forming a crystal form of zolpidem hemitartrate.
78. The method of clause 66, further comprising the step of crystallizing the zolpidem hemitartrate Form A from the solution.
79. The method of clause 66, further comprising the step of heating zolpidem hemitartrate to a temperature from about 70°C to about 150°C to form zolpidem hemitartrate Form C.
80. The process of clause 79, wherein the zolpidem hemitartrate is a form selected from the group of zolpidem hemitartrate polymorphs consisting of Forms A, D, E, F, and G, H, L.
81. The method of clause 66, further comprising the step of exposing zolpidem hemitartrate to vapors of ethanol to form zolpidem hemitartrate Form D.
82. The process of clause 81, wherein the zolpidem hemitartrate is a crystal form of zolpidem hemitartrate, selected from the group of crystal forms of zolpidem hemitartrate consisting of Form A and Form C.
83. The method of clause 66, further comprising the step of exposing zolpidem hemitartrate to water vapor at a relative humidity of about 100% to form zolpidem hemitartrate Form E.
84. The method of clause 83, wherein the zolpidem hemitartrate is a crystal form of zolpidem hemitartrate selected from the group of crystal forms of zolpidem hemitartrate consisting of Form A, Form C and Form D.
85. The method of clause 66, further comprising the step of exposing zolpidem hemitartrate to vapors of methanol to form zolpidem hemitartrate Form F.
86. The method of clause 85, wherein the zolpidem hemitartrate is a crystal form of zolpidem hemitartrate selected from the group of a crystal forms of zolpidem hemitartrate consisting of Form A and Form C.
87. The method of clause 66, further comprising the step of exposing zolpidem hemitartrate Form A to vapors of ethyl acetate to form zolpidem hemitartrate Form G.
88. The method of clause 66, further comprising the step of slurrying zolpidem hemitartrate Form A in ethanol to form zolpidem hemitartrate Form H.
89. The method of clause 66, further comprising:
   (a) dissolving zolpidem hemitartrate in a solvent mixture of methanol and water;
   (b) precipitating zolpidem hemitartrate from the solvent mixture; and,
   (c) isolating zolpidem hemitartrate, to form zolpidem hemitartrate Form L.
90. A process for preparing zolpidem hemitartrate Form C, comprising the steps of exposing zolpidem hemitartrate Form A to vapors of isopropyl alcohol.
91. A process for preparing zolpidem hemitartrate Form C, comprising the step of heating zolpidem hemitartrate to a temperature from about 70°C to about 150°C for a sufficient time to convert zolpidem hemitartrate to Form C.
92. A process for preparing zolpidem hemitartrate Form D, comprising the step of exposing zolpidem hemitartrate Form A to water vapor at a relative humidity from about 60% to about 100%.
93. A process for preparing zolpidem hemitartrate Form D, comprising the step of exposing Form C to water vapor at a relative humidity of about 100%.
94. A process for preparing zolpidem hemitartrate Form D, comprising the step of exposing zolpidem hemitartrate Form A to vapors of ethanol.
95. A process for preparing zolpidem hemitartrate Form D, comprising the step of exposing zolpidem hemitartrate Form C to vapors of ethanol.
96. A process for preparing zolpidem hemitartrate Form D, comprising the step of forming a slurry of zolpidem hemitartrate Form A in ethylacetate.
97. A process for preparing zolpidem hemitartrate Form D, comprising the step of forming a slurry of zolpidem hemitartrate Form A in acetone.
98. A process for preparing zolpidem hemitartrate Form D, comprising the step of granulating zolpidem hemitartrate Form A in isopropanol.
99. A process for preparing zolpidem hemitartrate Form C, comprising the step of forming a slurry of zolpidem hemitartrate Form A in isopropanol.
100. A process for preparing zolpidem hemitartrate Form D, comprising the step of granulating zolpidem hemitartrate Form A in butanol.
101. A process for preparing zolpidem hemitartrate Form E, comprising the step of exposing a solid form of zolpidem hemitartrate to water vapor at a relative humidity of about 100%.
102. A process for preparing zolpidem hemitartrate Form E, comprising the step of forming a slurry of a solid form of zolpidem hemitartrate in water.
103. A process for preparing zolpidem hemitartrate Form E, comprising the step of granulating a solid form of zolpidem hemitartrate in water.
104. A process for preparing zolpidem hemitartrate Form F, comprising the step of exposing a solid form of zolpidem hemitartrate to vapors of methanol.
105. A process for preparing zolpidem hemitartrate Form G, comprising the step of exposing zolpidem hemitartrate Form A to vapors of ethyl acetate.
106. A process for preparing zolpidem hemitartrate Form G, comprising the step of forming a slurry of zolpidem hemitartrate Form C in ethanol.
107. A process for preparing zolpidem hemitartrate Form G, comprising the step of forming a slurry of zolpidem hemitartrate Form C in methanol.
108. A process for preparing zolpidem hemitartrate Form G, comprising the step of granulating zolpidem hemitartrate Form C in ethanol.
109. A process for preparing zolpidem hemitartrate Form G, comprising the step of granulating zolpidem hemitartrate Form C in methanol.
110. A process for preparing zolpidem hemitartrate Form H, comprising the step of slurrying zolpidem hemitartrate Form A in ethanol.
111. A process for preparing zolpidem hemitartrate Form H, comprising the step of slurrying zolpidem hemitartrate Form A in methanol.
112. A process for preparing zolpidem hemitartrate Form H, comprising the step of granulating zolpidem hemitartrate Form A in ethanol.
113. A process for preparing zolpidem hemitartrate Form H, comprising the step of granulating zolpidem hemitartrate Form A in methanol.
114. A process for preparing zolpidem hemitartrate Form L, comprising the step of:
   (a) dissolving zolpidem hemitartrate in a solvent mixture of methanol and water;
   (b) precipitating zolpidem hemitartrate from the solvent mixture; and,
   (c) isolating zolpidem hemitartrate.
115. The process of clause 114, wherein the solvent mixture of methanol and water is at a ratio of about 13 parts methanol to about 1 part water.
116. Zolpidem hemitartrate having particles up to about 200 microns in size.
117. Zolpidem hemitartrate having particles up to about 50 microns in size.
118. A pharmaceutical composition comprising a therapeutically effective amount of zolpidem hemitartrate particles up to about 200 microns in size as measured by laser diffraction and, a pharmaceutically acceptable carrier.
119. The pharmaceutical composition of clause 118, wherein the zolpidem hemitartrate particles are selected from the group consisting of Form A, Form B, Form C, Form D, Form E, Form F, Form G, Form H and Form L.
120. A pharmaceutical composition comprising a therapeutically effective amount of zolpidem hemitartrate particles up to about 50 microns in size as measured by laser diffraction and, a pharmaceutically acceptable carrier.
121. The pharmaceutical composition of clause 120, wherein the zolpidem hemitartrate particles are selected from the group consisting of Form A, Form B, Form C, Form D, Form E, Form F, Form G, Form H and Form L.
122. Micronized zolpidem hemitartrate Form A having particles up to about 200 microns in size as measured by laser diffraction and an x-ray diffraction pattern having a peak at about 8.6 ±0.2 degrees two-theta.
123. The zolpidem hemitartrate of clause 122, further characterized by an x-ray diffraction pattern having peaks 6.7, 11.2, 15.4 and 17.3 ±0.2 degrees two-theta.

## Claims

1. A method for synthesizing zolpidem hemitartrate, comprising the steps of:
(a) forming a zolpidic acid halide from the zolpidic acid;
(b) reacting zolpidem acid halide, with dimethyl amine, to form zolpidem base;
(c) forming zolpidem hemitartrate salt from the zolpidem base.

2. The method of claim 1, wherein step (a) is carried out using toluene as solvent.

3. The method of claim 2, wherein the solvent further comprises DMF.

4. The method of any preceding claim, wherein the halide is chloride.

5. The method of claim 4, wherein the step of forming the acid chloride is performed using thionyl chloride, PCl₅ or POCl₃, preferably thionyl chloride.

6. The method of any preceding claim, wherein the reaction temperature of step (a) from about 15°C to about 28°C, preferably from about 18°C to about 22°C.

7. The method of any preceding claim, which further comprises following step (a) removing excess thionyl chloride by distillation.

8. The method of any preceding claim, wherein step (b) is carried out using toluene as solvent.

9. The method of any preceding claim, wherein the reaction temperature of step (b) from about -5°C to about +3°C, preferably from about -5°C to about 0°C.

10. The method of any preceding claim, wherein the dimethyl amine is introduced as a gas until the pH is from about 8.5 to about 9.5.

11. The method of any preceding claim, wherein step (a) and (b) are carried out in "one pot".

12. The method of any preceding claim, wherein step (c) is carried out using methanol as solvent.

13. The method of any preceding claim, which further comprising following step (c) isolating crystalline zolpidem hemitartrate.

14. The method of claim 13, wherein the crystalline zolpidem hemitartrate is selected from Forms A, B, C, D, E, F, G, H and L.

15. The method of claim 13, further comprising the step of heating zolpidem hemitartrate to a temperature from about 70°C to about 150°C to form zolpidem hemitartrate Form C.

16. The method of claim 13, further comprising the step of exposing zolpidem hemitartrate to vapors of ethanol to form zolpidem hemitartrate Form D.

17. The method of claim 13, further comprising the step of exposing zolpidem hemitartrate to water vapor at a relative humidity of about 100% to form zolpidem hemitartrate Form E.

18. The method of claim 13, further comprising the step of exposing zolpidem hemitartrate to vapors of methanol to form zolpidem hemitartrate Form F.

19. The method of claim 14, further comprising the step of exposing zolpidem hemitartrate Form A to vapors of ethyl acetate to form zolpidem hemitartrate Form G.

20. The method of claim 14, further comprising the step of slurrying zolpidem hemitartrate Form A to form zolpidem hemitartrate Form H.

21. The method of claim 13, further comprising:
(a) dissolving zolpidem hemitartrate in a solvent mixture of methanol and water;
(b) precipitating zolpidem hemitartrate from the solvent mixture; and,
(c) isolating zolpidem hemitartrate, to form zolpidem hemitartrate Form L.
